(19) Europäisches Patentamt
European Patent Office
Office européen des brevets

(11) Numéro de publication : **0 445 013 B1**

(12) # FASCICULE DE BREVET EUROPEEN

(45) Date de publication du fascicule du brevet : **08.06.94 Bulletin 94/23**

(21) Numéro de dépôt : **91400497.3**

(22) Date de dépôt : **25.02.91**

(51) Int. Cl.$^5$ : **C07D 307/52,** C07D 333/20, C07D 409/06, A61K 31/38, A61K 31/34

(54) **N-Cycloalkylalkylamines, leur procédé de préparation, leur utilisation comme médicament et leurs intermédiaires de synthèse.**

(30) Priorité : **26.02.90 US 484403**
**28.02.90 FR 9002494**

(43) Date de publication de la demande : **04.09.91 Bulletin 91/36**

(45) Mention de la délivrance du brevet : **08.06.94 Bulletin 94/23**

(84) Etats contractants désignés : **AT BE CH DE DK ES FR GB GR IT LI LU NL SE**

(56) Documents cités :
**EP-A- 0 297 782**
**EP-A- 0 298 703**

(73) Titulaire : **JOUVEINAL S.A.**
**Tour Maine Montparnasse**
**33 Avenue du Maine**
**F-75755 Paris Cedex 15 (FR)**

(72) Inventeur : **Aubard, Gilbert**
**7, chemin de la Savetière**
**F-91129 Palaiseau (FR)**
Inventeur : **Calvet, Alain**
**Résidence Ronsard,**
**3, rue de Gatine**
**F-94240 L'Hay les Roses (FR)**
Inventeur : **Defaux, Jean-Pierre**
**54, rue Sophie Rodriques**
**F-92500 Rueil (FR)**
Inventeur : **Gouret, Claude-Jean**
**34, rue Croix du Val**
**F-92190 Meudon (FR)**
Inventeur : **Grouhel, Agnès**
**2, rue des Peupliers**
**F-92190 Meudon (FR)**
Inventeur : **Hudspeth, James**
**3839 Northland St.**
**Newbury Park, California 91320 (US)**
Inventeur : **Jacobelli, Henri**
**65, Av. du Général de gaulle**
**F-91550 Paray Vieille Poste (FR)**
Inventeur : **Junien, Jean-Louis**
**36, Avenue Eiffel**
**F-92310 Sevres (FR)**
Inventeur : **Pascaud, Xavier**
**41, rue de Charenton**
**F-75012 Parijs (FR)**
Inventeur : **Roman, François**
**11, allée Pierre Fresnay**
**F-94400 Vitry Sur Seine (FR)**
Inventeur : **Yuan, Lin**
**426 N. Elisabeth Avenue AB,**
**Bat, B.**
**Mongerey Park, California 91754 (US)**

(74) Mandataire : **Bourgognon, Jean-Marie**
**Cabinet Flechner**
**22, Avenue de Friedland**
**F-75008 Paris (FR)**

EP 0 445 013 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouvelles N-cycloalkylalkylamines, leur procédé de préparation, leur utilisation comme médicaments et intermédiaires de synthèse.

Ces amines répondent à la formule générale (I) :

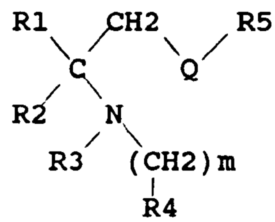

I

dans laquelle :

- R1 est un radical furyle ou un radical thiènyle, ou est un radical phényle sous réserve que Q représente un groupe cyclopropane 1,2 diyle

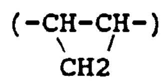

,

- R2 est alkyle inférieur,
- R3 est hydrogène ou alkyle inférieur,
- m a pour valeur 1 ou 2,
- R4 est cycloalkyle -CH(CH2)n dans lequel n est un nombre entier de 2 à 5.
- R5 est phényle pouvant être mono, di ou trisubstitué par des halogènes ou par des radicaux alkyle ou alkoxy inférieurs identiques ou différents, ou encore représente un radical thiényle,
- Q représente un groupe éthylène-1,2-diyle (-CH=CH-) ou un groupe cyclopropane 1,2-diyle

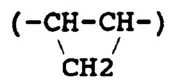

.

Dans cette description :

- par radical alkyle inférieur, on entend des radicaux linéaires ou ramifiés comprenant de 1 à 5 atomes de carbone,
- par halogène, on entend le brome, le fluor et le chlore à titre préféré,
- par alkoxy inférieur, des radicaux comprenant jusqu'à 3 atomes de carbone linéaires ou ramifiés et éventuellement substitués par des atomes d'halogène comme le fluor, les radicaux méthoxy étant toutefois les préférés.

En outre, les isomères optiques et/ou géomètriques consécutifs aux centres de dissymétries des cycloalkylalkylamines (I) font partie intégrante de l'invention. Il en est de même des sels d'addition des amines (I) avec les acides minéraux ou organiques pharmaceutiquement acceptables ainsi que leurs éventuels solvats.

A titre d'acides fréquemment utilisés pour la préparation des sels d'addition on cite de façon non limitative les acides acétique, benzènesulfonique, camphosulfonique, citrique, éthanesulfonique, fumarique, bromhydrique, chlorhydrique, lactique, maléïque, malique, méthanesulfonique, mucique, nitrique, pamoïque, phosphorique, salicylique, stéarique, succinique, sulfurique, tartrique.

Etudiées chez l'animal, les cycloalkylalkylamines (I) de l'invention et leurs sels se montrent faiblement toxiques et révèlent à la fois :

- une activité psychotrope montrée par leur aptitude à inhiber les crises convulsives provoquées par la picrotoxine,
- une activité inhibitrice de l'effet ulcérogène de la cystéamine au niveau gastro-duodénal, propriété imputée à l'affinité particulière des composés pour les récepteurs sigma présents dans ces organes.

Aussi, les composés de l'invention sous forme de médicaments justifient d'une utilité indéniable et dans

ces indications on préfère que l'une au moins des conditions suivantes soit satisfaite :

R1 est un radical thiènyle,
R2 est alkyle inférieur comprenant de 1 à 3 atomes de carbone et notamment est éthyle,
R3 est méthyle,
m a pour valeur 1,
R4 est cycloalkyle CH(CH2)n et dans lequel n a pour valeur 2,
R5 est phényle,
Q est le groupe éthylène-1,2-diyle (-CH=CH-) ou le groupe cyclopropane-1,2-diyle

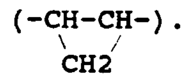

L'invention vise aussi un procédé de préparation des amines (I) qui consiste comme il est montré au schéma 1 :

- pour préparer un composé (II) de formule générale (I) et dans laquelle R3 est l'hydrogène et R1, R2, m, R4, R5 et Q ont les significations générales précédemment énoncées,
  i) à acyler une amine (V) de formule :

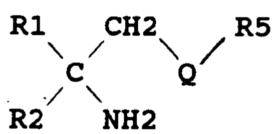

V

dans laquelle R1, R2, R5 et Q sont tels que précédemment définis, par un réactif (VI)

(R4-[CH2)m-1]-CO)pZ1 VI

dans lequel :

R4 et m ont les significations définies plus haut,
p a pour valeur 1 ou 2
Z1 est hydroxyle (-OH) ou halogène comme le chlore ou le brome lorsque p = 1 et est un atome d'oxygène lorsque p=2,
pour obtenir un carboxamide intermédiaire (IV)

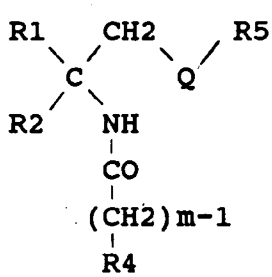

IV

que l'on réduit par un hydrure métallique (Hm) de formule

M1(t) M2 H(r) Rx(s) (Hm)

dans laquelle

M1 représente un atome alcalin comme le lithium ou le sodium et dont l'indice (t) a pour valeurs 0 ou 1,
M2 est un élément du groupe III de la classification périodique des éléments et de préférence le bore ou l'aluminium,
(r) est l'indice représentatif du nombre d'atomes d'hydrogène de l'hydrure et qui a pour valeurs 1, 2, 3 ou 4,
Rx est un groupe carbonitrile, un radical alkyle ou alkoxy inférieur dont l'indice représentatif (s) a pour valeurs 0, 1, 2 ou 3,

Les indices présentés ci-dessus répondant pour ces hydrures à la relation : (r) + (s) - (t) = 3

et dans lesquels on préfère pour réduire les composés (IV) les hydrures (Hm.2) dans lesquels M2 est l'aluminium ou encore le bore lorsque (r) a pour valeur 3 et (s) et (t) ont pour valeur 0,

en une cycloalkylalkylamine (II) de formule générale (I) dans laquelle R3 est l'hydrogène

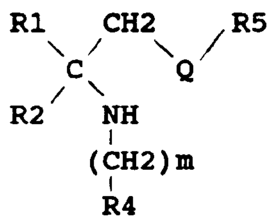

II

ii) à alkyler une amine (V) par un halogénure d'alkyle R4-(CH2)m-Z2 dans lequel R4 et m ont les significations déjà citées et Z2 est un halogène tel que le chlore, le brome ou l'iode.

- et pour préparer une cycloalkylalkylamine (III) de formule générale (I) dans laquelle R3 est alkyle inférieur et R1, R2, m, R4, R5 et Q ont les significations générales déjà précisées,

i) à procéder à une alkylation réductrice d'un composé (II) de l'invention qui consiste à le faire réagir avec un aldéhyde R6-CHO dans lequel R6 est l'homologue carboné immédiatement inférieur au radical R3 à introduire (R3 = CH2-R6), et avec un agent réducteur comme un hydrure métallique ou organométallique (Hm.3) de formule (Hm) déja définie et dans laquelle plus particulièrement M2 est le bore et, de façon préférée Rx est un groupe carbonitrile,

ii) ou à acyler une amine (VII)

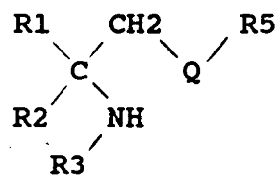

VII

dans laquelle R1, R2, R3, R5 et Q ont les significations précisées pour (I), par un halogénure d'acyle de formule R4-[(CH2)m-1]COZ4 dans lequel R4 et m ont les significations déjà définies et Z4 est un halogène et plus particulièrement le chlore ou le brome, pour obtenir le N-carboxamide intermédiaire (VIII)

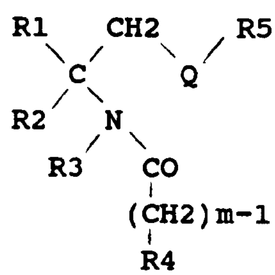

VIII

qui est réduit par un hydrure métallique (Hm.2) précédemment défini, en une cycloalkylalkylamine (III) de l'invention,

iii) ou, à faire réagir sur un intermédiaire amino nitrile (IX)

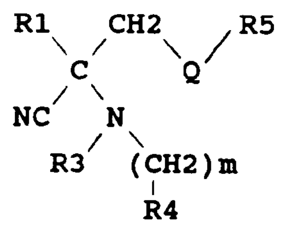

IX

dans lequel R1, R3, m, R4 , R5 et Q sont tels que définis pour (I), un réactif organomagnésien R2MgZ3 dans lequel R2 est alkyle inférieur et Z3 halogène comme le chlore, le brome ou l'iode, pour obtenir une cycloalkylalkylamine (III).

iiii) ou encore à alkyler une amine (VII) précédemment définie par un halogénure d'alkyle R4-(CH2)m-Z2 dans lequel R4 et m ont les significations définies pour (I) et Z2 est un halogène tel que le chlore le brome ou l'iode.

SCHEMA 1

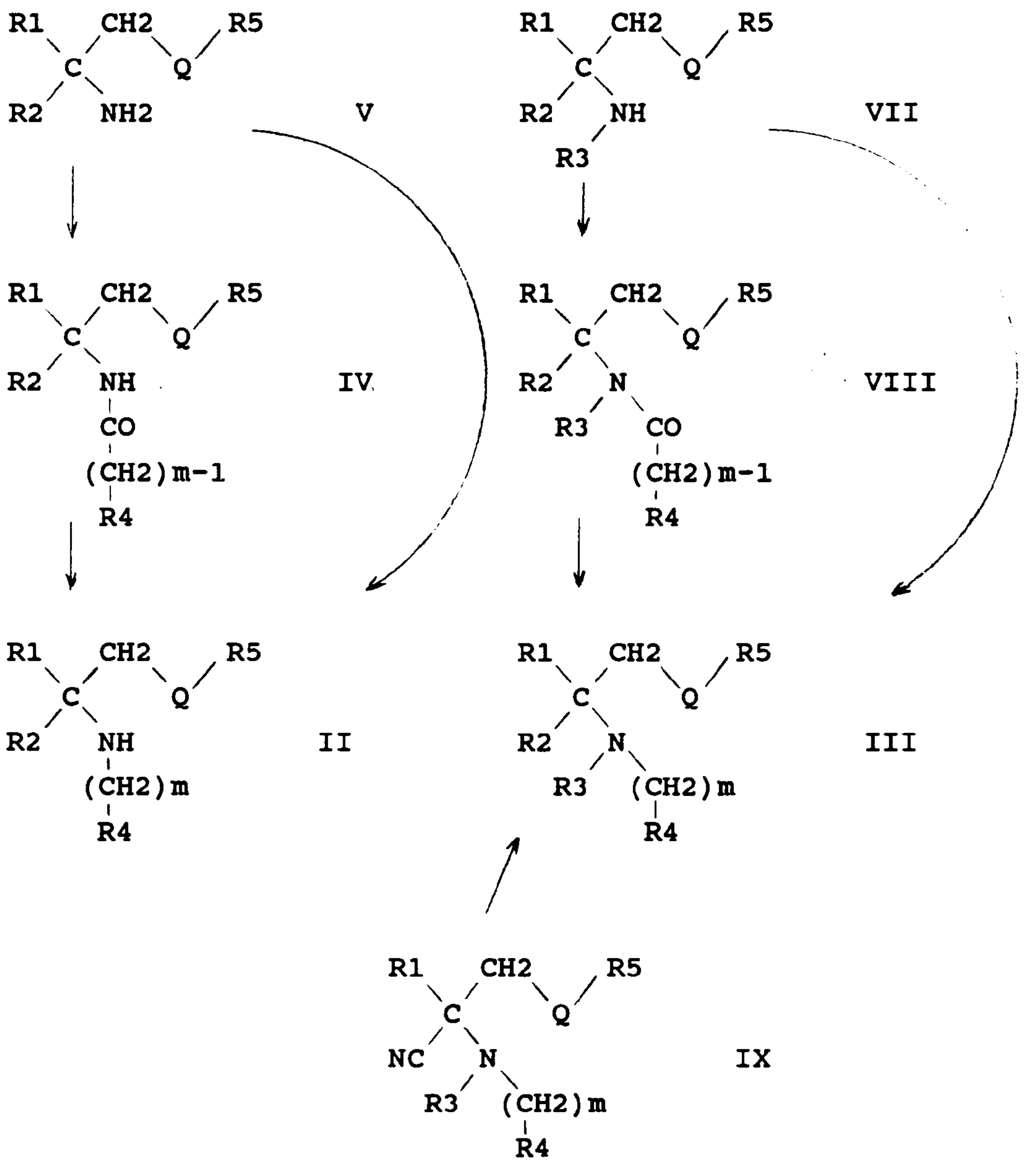

V VII IV VIII II III IX

A la demande de brevet européen n° 0 298 703 il est décrit des dérivés du thiophène de formule :

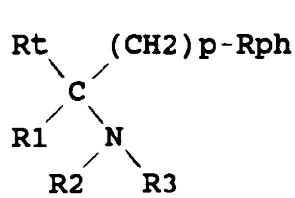

dans laquelle, au sens le plus large,

- Rt est un radical thiényle,
- R1, R2 et R3 représentent des radicaux alkyle inférieur,

- Rph est un radical phényle éventuellement substitué,
- p a pour valeurs 1, 2 ou 3, et, dans laquelle, pour les composés préférés,
- R1 est un radical éthyle,
- R2 et R3 sont des radicaux méthyle,
- Rph est un radical phényle, 3,4-diméthoxyphényle, 3,4,5-triméthoxyphényle ou 4-chlorophényle,
- p a pour valeurs 1 ou 3.

Les composés sont présentés comme étant peu toxiques et ayant un effet régulateur de la motilité du tractus gastro intestinal caractérisé par un effet stimulant sur un tractus d'activité ralentie et, inversement, par un effet inhibiteur sur un tractus hyperactif.

Différents de par leur structure chimique, notamment par la nature de la chaine carbonée reliant les deux sites aromatiques qui sont dénommés Rt et Rph dans la formule précédente et par la nature des substituants de la fonction aminée, les composés de la demande européenne différent des cycloalkylalkylamines (I) de la présente invention par leurs propriétés. Essentiellement il n'est pas rapporté d'activité de type psychotrope pour les composés de la demande n° 0 298 703, alors que l'activité pharmacologique des amines (I) permet d'envisager, entre autres, leur application dans le traitement des affections neuro-psychiques. De même il n'est fait aucune mention de l'activité des composés de cet art antérieur à agir sur les lésions ulcéreuses du tractus gastro intestinal.

Comme il a été précédemment exposé les composés intermédiaires qui permettent la préparation des produits (I) de l'invention sont essentiellement les dérivés de structure (V) (VII) et (IX).

Le procédé de préparation des composés (V) consiste à alkyler un composé R1-CH2-W dans lequel R1 est tel que défini pour (I) et W est un radical carbonitrile (-CN) ou carboxyle (-COOH) par un halogènure d'alkyle de formule R2Z6, R2 étant tel que défini pour (I) et Z6 étant un halogène, pour obtenir, pour W = -COOH un acide de formule (XV) R1(R2)-CH-COOH, et, pour W = -CN, obtenir un nitrile de formule (XVI) R1(R2)-CH-CN qui est hydrolysé en acide (XV), puis à alkyler l'acide (XV) par un réactif (XIII) de formule Z5-CH2-Q-R5, Z5 étant un halogène ou un radical alkylsulfonyloxy, R5 et Q tels que définis pour (I) pour obtenir les acides (XIV) R1(R2)C(COOH)CH2-Q-R5 puis à préparer, comme il est montré au schéma 2, les isocyanates (X) par la réaction de Curtius à partir de ces acides

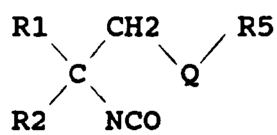

X

dans lesquels R1, R2 , R5 et Q ont les significations précisées pour (I), et finalement à hydrolyser leur fonction isocyanate dans des conditions identiques à celles précisées plus loin pour les composés (X') pour obtenir les composés amines primaires (V).

Il est aussi possible pour obtenir ces derniers d'utiliser un procédé adapté de celui décrit à la demende de brevet européen n° 0 298 703 déja citée et qui consiste tel que montré au schéma 2 à faire réagir une cétone R1-CO-R2 avec le formamide et l'acide formique pour préparer le formamide N-substitué de formule R1(R2)CH-NH-CHO que l'on déshydrate par de l'oxychlorure de phosphore pour obtenir un isonitrile correspondant de formule R1(R2)CH-NC qui, alkylé par un réactif (XIII) conduit à l'intermédiaire isonitrile (X') de formule

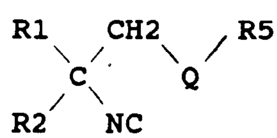

X'

qui est hydrolysé par un acide fort minéral comme l'acide chlorhydrique qui est préféré et ce dans un solvant aqueux contenant une cétone de faible poids moléculaire comme l'acétone en quantité suffisante pour obtenir un milieu réactionnel homogène, afin d' obtenir un composé amine primaire (V).

Pour préparer les intermédiaires de formule (VII), lorsque R3 est méthyle, le procédé préféré consiste à réduire par un hydrure métallique ou organo métallique (Hm.1) de formule (Hm) déja définie et dans laquelle M2 est de préférence l'aluminium, la fonction isocyanate d'un intermédiaire (X) ou la fonction isonitrile d'un

intermédiaire (X′) et, lorsque R3 est alkyle inférieur soit à faire réagir sur un composé (V) déja décrit un halogénure d'alkyle Z5R3 dans lequel Z5 est le chlore, le brome ou l'iode,

ou bien à acyler un intermédiaire (V) par un halogénure d'acyle R6COZ6 dans lequel R6 est l'homologue inférieur d'un atome de carbone à R3 (R3 = CH2-R6) pour obtenir un intermédiaire de formule :

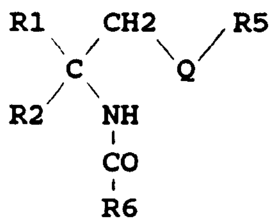

puis à le réduire par un hydrure métallique (Hm.2) tel que précédemment décrit.

Le procédé de préparation des amino nitriles (IX) consiste à préparer un amino nitrile (XII) : R1(NC)CH-N(R3)(CH2)m-R4, à partir d'un aldéhyde R1-CHO, d'une amine secondaire HN(R3)(CH2)m-R4 et d'un cyanure de métal alcalin selon la réaction de Strecker et par une technique inspirée de celle décrite par S.F. Dyke et coll., Tetrahedron 1975, 31, p. 1219, puis à alkyler le dérivé (XII) par un réactif Z5-CH2-Q-R5 (XIII) dans lequel R5 a les valeurs définies pour (I) et Z5 est le chlore, le brome ou l'iode ou un radical alkylsulfonyloxy.

Cette alkylation est réalisée en préparant d'abord l'anion de l'amino nitrile (XII) par action dans un solvant inerte comme le THF d'une base organo-métallique forte appropriée. Le N,N-diisopropyl amide de lithium est préféré (LDA). Il est préparé "in situ" à partir de quantités équimoléculaires de N,N-diisopropyl-amine et de butyllithium.

Après réaction du composé (XII) durant 1 à 2 h entre 20 et 100°C on ajoute à l'anion le réactif (XIII) et laisse le milieu réactionnel de 1 à 2 h à la température ambiante voisine de 20°C pour obtenir l'intermédiaire (IX) qui est purifié.

SCHEMA 2

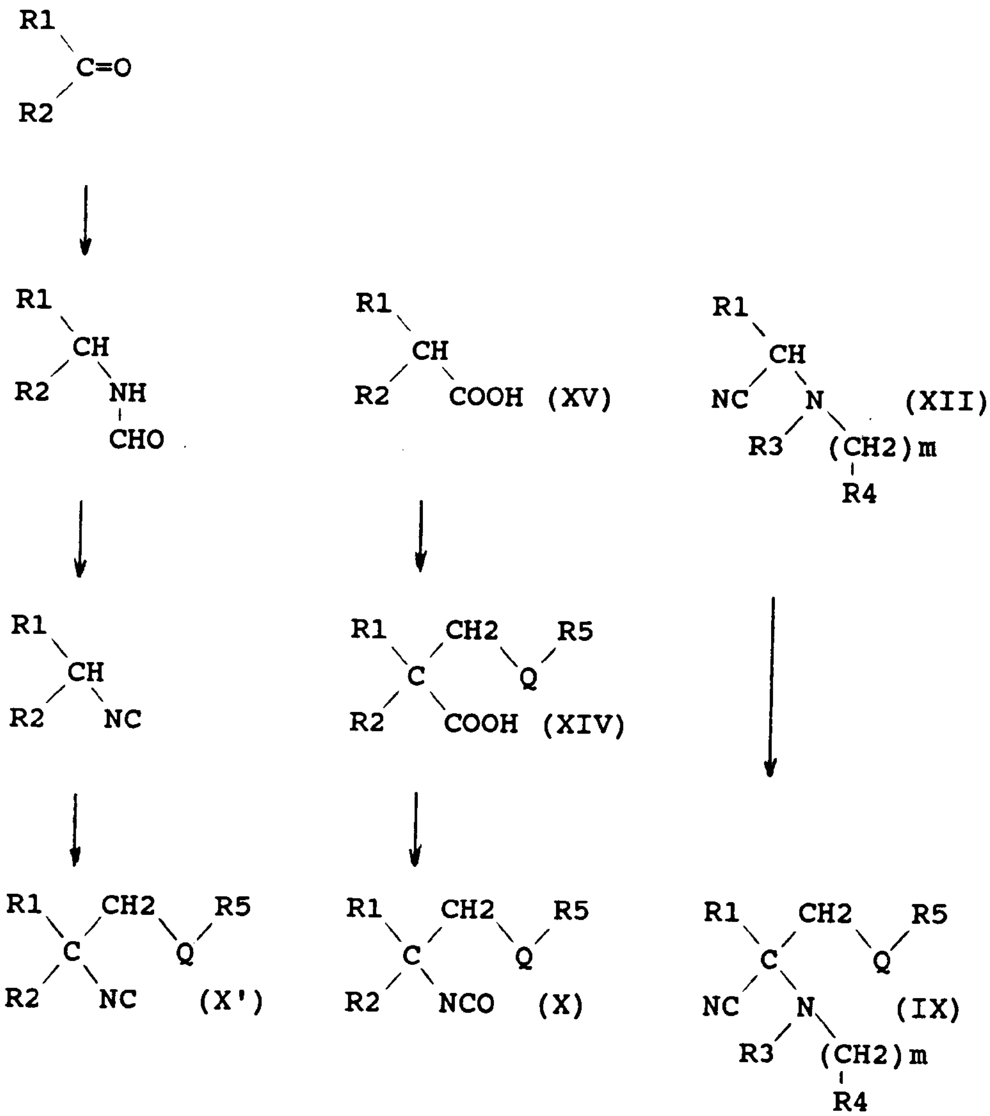

(XV) (XII) (XIV) (X') (X) (IX)

De façon explicite la préparation de composés de l'invention (II) et (III) de formule générale (I) est développée dans ce qui suit :

a) lorsque le procédé consiste à acyler un composé (V) par un réactif (VI) [R4-[(CH2)m-1]-CO]pZ1 pour obtenir un intermédiaire N-carboxamide (IV) qui est ensuite réduit.

Lorsque le réactif (VI) est un halogénure d'acide (p = 1, Z1 = halogène) la réaction préférée s'effectue en milieu monophasique dans le toluène ou plus favorablement le dichlorométhane, et consiste à ajouter dans une solution contenant une mole de dérivé à acyler de 1,0 à 1,5 mole d'une base de type amine qui est généralement la triéthylamine, puis à ajouter le réactif (VI) en quantité équimoléculaire à la triéthylamine. La solution est ensuite maintenue de 3 à 48 h à une température comprise entre 15 et 30°C de façon à obtenir la réaction la plus complète possible.

Et, lorsque le réactif d'acylation (VI) est un anhydride d'acide (p = 2; Z1 = oxygène), la réaction, lorsque le point d'ébullition de l'anhydride est inférieur à 140°C, peut s'effectuer sans solvant, en faisant réagir le composé (V) dans un large excès et à la température de reflux du réactif (VI). La méthode préférée consiste cependant à réaliser la réaction en utilisant comme solvant la pyridine et en faisant réagir pour une mole de composé à acyler, de 1 à 5 mole d'anhydride. Couramment l'utilisation de 1,2 à 1,8 mole d'anhydride au reflux de la pyridine durant 1 à 3 heures conduit à des résultats convenables.

La méthode d'acylation préférée de (V) lorsque le réactif (VI) est un acide carboxylique (p = 1 ; m = 1 ou 2; Z1 = OH) consiste à préparer, in situ, un anhydride pouvant être mixte, à partir de cet acide carboxylique, puis à acyler l'intermédiaire (V) par cet anhydride. Favorablement, la réaction est effectuée dans des solvants apolaires anhydres de la classe des éthers oxydes. Le tetrahydrofurane est préféré et on forme d'abord l'anhydride mixte à une température comprise entre - 40 et 0°C en ajoutant pour une mole d'acide (VI) de 1,0 à 1,5 mole d'amine tertiaire comme la N-méthylmorpholine puis de 0,9 à 1,2 mole de chloroformiate d'isobutyle.

On ajoute ensuite une mole d'intermédiaire (V) à acyler et laisse la réaction se développer de 1 à 48 heures à une température comprise entre 0 et 60°C. Habituellement, le résultat de la réaction est satisfaisant à une température comprise entre 10 et 25°C après une durée de 10 à 20 heures.

Des méthodes alternatives peuvent employer d'autres agents de déshydratation énumérés par exemple dans "Advanced Organic Chemistry", J. March. Ed. Wiley 1985. p. 349. Ainsi la réaction a été réalisée avec pour agent de déshydratation en milieu anhydre le dicyclohexyl-carbodiimide et, plus particulièrement avec l'acide formique, en utilisant le N-N'-carbonyl diimidazole.

La réduction des intermédiaires N-carboxamides est réalisée par des hydrures métalliques ou organométalliques (Hm.2) précédemment définis, de façon appropriée à la réduction spécifique de la fonction carboxamide.

A cet effet, on utilise favorablement l'hydrure de lithium aluminium ou l'hydrure d'aluminium qui est préféré. Les réactions sont effectuées dans des solvants inertes aux réactifs utilisés tels que dans des éthers comme par exemple l'éther diéthylique, le 1,2-diméthoxy éthane ou le tétrahydrofurane (THF) qui est préféré.

Egalement de façon préférée, l'agent réducteur utilisé, l'hydrure d'aluminium peut être préparé "in situ" à partir d'halogènures d'aluminium et d'hydrures métalliques comme il est par exemple décrit dans "Réduction with complex metal hydrides" - N.G. Gaylord., 1956, Ed. Interscience - p. 6 à 8, et 51 à 53.

Avantageusement, la réaction de réduction dans le THF d'une mole d'intermédiaire (IV) ou (VIII) consiste dans un premier temps à préparer "in situ" l'hydrure d'aluminium par réaction sur 0,75 à 2 mole de chlorure d'aluminium, de 2,25 à 6 mole d'hydrure de lithium aluminium, ces réactifs étant utilisés dans un rapport moléculaire voisin de 1 pour 3, puis à introduire à une température comprise entre - 10 et + 30°C l'intermédiaire N-carboxamide, à laisser la réaction de réduction se développer de 1 à 24 h à la même température, puis à décomposer le complexe réduit obtenu et à isoler les composés de l'invention (II) ou (III) par les méthodes appropriées.

Le plus couramment, les réductions sont effectuées à une température comprise entre 10 et 20°C durant 2 à 6h.

Tel qu'il a été précédemment décrit pour l'utilisation du réactif (VI) lorsqu'il est un halogènure d'acide, la réaction s'applique également à l'acylation des intermédiaires (VII) par les réactifs R4-[(CH2)m-1]-CO-Z4 dans lesquels Z4 est le chlore ou le brome, afin de préparer les carboxamides intermédiaires (VIII) qui, réduits tel qu'indiqué, permettent d'obtenir les cycloalkylalkylamines (III) de l'invention.

b) Lorsque le procédé consiste à N-alkyler un intermédiaire (V) ou (VII) par un halogénure d'alkyle R4-(CH2)m-Z2 déjà décrit et dans lequel Z2 est le chlore, le brome ou l'iode la réaction est réalisée dans des solvants inertes aux réactifs comme par exemple le toluène et l'acétonitrile, et en faisant réagir une mole d'intermédiaire (V) ou (VII) avec de 0,5 à 1,5 mole d'halogènure.

D'une manière préférée, on utilise de 0,80 à 1,20 mole de dérivé où l'halogène est le brome ou l'iode et optionnellement on ajoute une base, organique ou minérale, pour favoriser la réaction qui consiste à chauffer le milieu réactionnel à une température comprise entre 20 et 110°C et ce durant de 2 à 5 h, les produits étant ensuite isolés et purifiés par les méthodes habituelles, notamment par chromatographies.

c) Lorsque le procédé consiste à pratiquer une N-alkylation réductrice pour obtenir un composé de l'invention (III) à partir d'un composé (II) et d'un aldéhyde R6-CHO il peut être pratiqué différentes techniques dont l'essentiel est présenté dans "Advanced Organic Chemistry", J. March - 3ème Ed. - Wiley 1985 - p. 798-800.

Avantageusement, pour les divers réactifs carbonylés employés, excepté le formaldéhyde, la réaction peut être effectuée dans un solvant protique anhydre comme un alcool inférieur tel que le méthanol ou l'éthanol, en faisant réagir un mole de composé (II) avec 1,5 à 10 mole de composé carbonylé en présence d'un catalyseur acide anhydre comme l'acide acétique, l'acide p-toluéne-sulfonique.

La réaction est ainsi réalisée entre 30 minutes et 8 heures à une température de milieu comprise entre 20°C et celle de reflux du solvant. Puis, à une température voisine de 20°C on ajoute un hydrure réducteur de formule (Hm.3) précédemment définie et dans lesquels on préfère le borohydrure de sodium ou le cyanoborohydrure de sodium à raison de 0,5 à 2,5 mole par mole de composé (II) engagé.

Particulièrement, quand le procédé consiste en l'alkylation d'un composé (II) par le formaldéhyde pour obtenir un produit de l'invention (III) dans lequel R3 est méthyle, on pratique avantageusement la méthode décrite dans J. Med. Chem. 1982, 25, 4, p. 446-51, qui consiste à faire réagir dans l'acétonitrile, le formaldéhyde en solution aqueuse en présence de cyanoborohydrure de sodium.

d) Lorsque le procédé pour préparer un composé de l'invention (III) consiste à faire réagir un amino-nitrile (IX) avec un réactif organomagnésien R2MgZ3, la substitution de la fonction carbonitrile par le radical R2 est réalisée selon une méthode inspirée de celle décrite par N.J. Léonard et coll., J. Am. Chem. Soc., 1956, 78, p. 1986 et 1957, 79, p. 5279. Elle est effectuée dans les éthers comme l'éther diéthylique, le méthyl-t.butyl éther, les éthers di-isopropylique ou dibutylique ou encore le tétrahydrofurane qui est préféré, et consiste, pour une mole de composé (IX), à faire réagir 1,5 à 6 mole de dérivé organo magnésien à une température comprise entre 5 et 50°C et ce pendant 30 minutes à 12 heures.

Avantageusement, la méthode consiste à ajouter à une température comprise entre 10 et 20°C, 1 mole de composé (IX), généralement en solution dans le THF, à 4 à 5 mole du composé organomagnésien également en solution dans le THF. La réaction est poursuivie durant de 2 à 5 heures à la même température puis le complexe obtenu décomposé par addition de solution aqueuse de chlorure d'ammonium. Après traitements, le composé de l'invention (III) est isolé et purifié.

Les modes opératoires qui suivent illustrent la préparation de dérivés intermédiaires et des composés (I) de l'invention.

Les composés sont, selon les réactions effectuées, soit obtenus tels quels dans un état de pureté satisfaisant, soit purifiés par des techniques appropriées indiquées dans les exemples comme la cristallisation, la distillation sous vide ou encore la chromatographie sur colonne. Dans ce dernier cas on utilise favorablement la technique dite de "chromatoflash" sur un support de silice (marque "Merck", produit Kieselgel 60, granulométrie 230 à 400 mesh).

Par ailleurs, la pureté, l'identité et les caractéristiques physico-chimiques des produits préparés sont rapportées et déterminées par :

- leur point d'ébullition sous la valeur du vide lors de leur distillation,
- leur point de fusion, déterminé par la méthode du tube capillaire et dont la valeur indiquée n'est pas corrigée,
- la chromatographie sur couches minces (CCM) de silice (plaques prêtes à l'emploi, "Merck" ref. 60 F 254) dont la technique est brièvement rappelée :

les produits à étudier sont déposés sur la plaque à raison de 100 mcg environ puis élués de façon ascendante par des solvants ou leurs mélanges qui sont énumérés ci-après, les proportions respectives étant indiquées en volumes pour volumes :

| | | | | | | |
|---|---|---|---|---|---|---|
| réf. | S.A | - | hexanes | 100 / | acétate d'éthyle | 10 |
| | S.B | - | " | 40 / | " | 10 |
| | S.C | - | " | 20 / | " | 10 |
| | S.D | - | dichlorométhane | 99 / | triéthylamine | 1 |

Après développement, les chromatogrammes sont observés sous lumière ultra violette de 254 nm de longueur d'onde et/ou après révélation colorée par pulvérisation du réactif de Dragendorff ou du réactif à la tolidine. Les Rf observés ainsi que les références des solvants d'élution utilisés sont indiqués dans les exemples.

- l'analyse centésimale élémentaire dont les résultats, conformes aux normes admises ne sont pas reportés, mais sont signalés être effectués par la représentation de l'élément dosé,
- la spectrographie infra rouge des composés en pastilles dans le KBr ou sous forme de films entre deux fenêtres de NaCl ou encore en suspension dans le nujol (R) ou encore en solution dans le CCl4; les absorbtions les plus intenses sont rapportées par la valeur de leur longueur d'onde en cm-1
- la résonance magnétique nucléaire du proton (RMN) est étudiée à 60 ou 90 MHz, les produits étant solubilisés dans le deutérochloroforme. L'aspect des signaux, leur déplacement chimique exprimé en p.p.m par rapport au tétraméthylsilane utilisé comme référence interne sont indiqués. Les protons dits "échangeables" après addition d'oxyde de deutérium sont également signalés.

Enfin, divers réactifs ou solvants peuvent être indiqués sous leur forme abrégée courante, entre autres exemples, THF pour le tétrahydrofurane.

PARTIE EXPERIMENTALE - PREPARATIONS

A- réactifs de formule (XIII) : Z5-CH2-Q-R5

A-1/ trans 1-mésyloxyméthyl-2-phényl-cyclopropane

(R5 = C6H5; Z5 = CH3-SO2-O; Q = cyclopropane-1,2-diyle)

A 230 ml (230 mmol) d'une solution de borane dans le THF on ajoute goutte à goutte sous atmosphère d'azote une solution de 25,0g d'acide trans 2-phényl-1-cyclo-propanecarboxylique (154 mmol) dans 100 ml de THF. La solution est portée au reflux durant 3 heures puis on ajoute lentement 130 ml de solution NaOH 2N. Le mélange est agité durant 30 minutes puis extrait à l'éther ; les phases éthérées sont déshydratées sur MgSO4 puis concentrées sous vide pour obtenir le produit résiduel brut : 20,8 g. L'intermédiaire trans 1-hydroxyméthyl-2-phényl-cyclopropane est purifié par distillation sous vide.

Poids = 18,2 g Rdt = 80 % Eb = 90-97°C/35 Pa

Dans 100ml de dichlorométhane on dissout 9,2 g (66 mmol) du produit précédemment obtenu et 13,8 ml (99 mmol) de triéthylamine. Sous atmosphère d'azote à - 10°C on ajoute goutte à goutte 5,6 ml (73 mmol) de chlorure de méthanesulfonyle. Le mélange est agité 15 minutes à -10°C puis lavé par extractions à l'eau, à l'acide chlorhydrique dilué froid, puis avec une solution saturée en NaHCO3, une solution saturée en ClNa puis séchée sur MgSO4 à 0°C.

Le solvant est éliminé par distillation sous vide à une température inférieure à 10°C, le produit instable est dissout dans le THF anhydre et engagé tel quel dans la suite des préparations.

A-2/ trans 1-bromométhyl-2-phényl-cyclopropane.

(R5 = C6H5 ; Z5 = Br ; Q = cyclopropane-1,2-diyle)

On ajoute 61,0 g (0,34 mol) de N-bromosuccinimide à 300 ml de chlorure de méthylène. La suspension est refroidie à 0°C et sous atmosphère d'azote on ajoute 29,4 ml (0,41 mol) de sulfure de diméthyle ; le mélange est ensuite agité 30 minutes puis refroidi à -25°C on ajoute alors goutte à goutte une solution de 33,6 g (0,23 mol) de trans 1-hydroxyméthyl-2-phényl-cyclopropane.

Le mélange est agité 6 heures à 0°C puis 16 heures à 25°C; après dilution par 250 ml d'hexanes le mélange est précipité dans 250 ml d'eau glacée. La phase organique est lavée avec une solution saturée en chlorure de sodium puis séchée sur MgSO4. Les solvants sont éliminés par distillation sous vide et le résidu purifié par distillation.

Poids = 40,8g Rdt = 85% Eb = 72°C/25 Pa.

A.3. - 1-(2-thiènyl)-3-chloroprop-1-ène

(R5 = 2-thiènyle ; Q = -CH=CH- ; Z5 = Cl)

- 1er stade : 104,06 g d'acide malonique (1,0 mol), 56,07 g (0,50 mol) de 2-thiophène carboxaldéhyde, 250 ml de pyridine et 5ml de pipéridine sont chauffés au bain marie 2 heures puis au reflux durant 5 minutes. Après refroidissement, la solution est précipitée dans l'eau et traitée par un excès d'acide chlorhydrique (250 ml de solution concentrée à 37 %) pour précipiter le produit qui est ensuite filtré puis recristallisé dans un mélange éthanol-eau pour obtenir l'acide 2-thiènylacrylique purifié.
  Poids = 42,38 g Rdt = 58 % F = 143-144°C
- 2ème stade : 37,34 g (0,24 mol) de l'acide précédent, 30 ml (0,24 mol) de complexe BF3-éther dans 310 ml de méthanol sont chauffés au reflux durant 6 heures. La solution refroidie est précipitée dans l'eau puis extraite au dichlorométhane. Les phases d'extraction organiques sont réunies, lavées par une solution saturée en NaHCO3 puis avec une solution saturée en NaCl puis déshydratées sur MgSO4. Le résidu solide de couleur brune qui est obtenu après élimination des solvants par distillation est recristallisé dans l'hexane pour obtenir le 2-thiènylacrylate de méthyle purifié.
  Poids = 32,65 g Rdt = 81 % F = 46-47°C
- 3ème stade : à un mélange de 5,28 g (39,6 mmol) de chlorure d'aluminium et de 40 ml d'éther diéthylique refroidi à -10°C et sous atmosphère d'azote on ajoute lentement sous agitation une suspension de 4,51 g (118,9 mmol) d'hydrure de lithium aluminium dans 150 ml de THF.

  Une solution de 10,0 g (59,45 mmol) d'ester méthylique précédent dans 50 ml de THF est lentement ajoutée à -10°C puis la solution est agitée à la même température durant une heure et demie. Les complexes de la solution sont décomposés par addition d'une solution d'acide sulfurique 3M et le mélange est extrait à l'éther. Les phases éthérées réunies sont lavées par une solution saturée en NaHCO3

puis par une solution saturée en NaCl et déshydratées sur MgSO4. Par évaporation de l'éther sous vide on obtient 7,83 g (94 %) de produit résiduel sous forme d'une huile brune. Le 1-(2-thiènyl)-prop-1-èn-3-ol brut instable à température ambiante est conservée à une température inférieure à O°C.

- 4ème stade : dans un mélange de 39,53 g (296 mmol) de N-chlorosuccinimide dans 180 ml de dichlorométhane anhydre à une température de 0°C on ajoute lentement 21,74 ml (296 mmol) de sulfure de diméthyle. Le mélange est refroidi à -10°C et une solution de 11,86 g (84,6 mmol) de l'alcool précédent dans 50 ml de dichlorométhane est ajoutée.

La température de la solution est ramenée à 0°C et y est maintenue 2 heures. Le mélange est dilué par 100 ml d'hexanes puis précipité dans 200 ml d'eau glacée. La phase organique est séparée et la phase aqueuse réextraite à l'éther. Les phases éthérées réunies sont lavées puis déshydratées. L'éther est éliminé par distillation sous vide, on obtient sous forme d'huile marron instable le 1-(2-thiènyl)-3-chloro-prop-1-ène brut qui est utilisé tel quel.

Poids = 12,06 g     Rdt = 94 %

A.4 - 1-(3-thiènyl)-3-chloro-prop-1-ène

(R5 = 3-thiènyle ; Q = -CH=CH- ; Z5 = Cl)

L'intermédiaire est obtenu à partir de 3-thiophène-carboxaldéhyde selon le procédé décrit à l'exemple précédent.

- 1er stade : acide 3-thiènylacrylique
  F = 146°C (éthanol/eau)
- 2ème stade : 3-thiènylacrylate de méthyle
  F = 49°C (hexanes)
- 3ème stade : 1-(3-thiènyl)-prop-1-èn-3-ol huile non purifiée
- 4ème stade : 1-(3-thiènyl)-3-chloro-prop-1-ène solide blanc amorphe non purifié

A.5 - trans-1-chlorométhyl-2-(2-thiényl)-cyclopropane

(R5 = 2-thiènyle ; Q = cyclopropane-1,2-diyle, Z5 = Cl)

- 1er stade : Dans un appareil anhydre et sous atmosphère d'azote, on introduit 33,9 ml d'alcool t. butylique deshydraté et 25 ml de THF. A 0°C on introduit sous agitation 144 ml d'une solution 2,5 M de butyl lithium dans l'hexane. Après retour à température ambiante, le mélange est maintenu sous agitation 30 minutes.

  On ajoute ensuite 20,0 g de 2-thiénylacrylate de méthyle purifié (préparé comme il est indiqué aux stades 1 et 2 du composé A.3) en solution dans 125 ml de THF.

  Le mélange est agité 3 heures puis précipité dans 600 ml d'eau glacée puis extrait par 3 fois 100 ml d'acétate d'éthyle. Les phases organiques réunies sont séchées sur MgSO4 puis l'acétate d'éthyle éliminé par distillation sous vide. Le résidu huileux marron représente 26,0 g.

  Le 2-thiénylacrylate de t.butyle est purifié par distillation sous pression réduite.

  Poids : 21,3 g     Rdt : 85 %     Eb = 82-90°C/4 pa
- 2ème stade : A une suspension agitée de 74,8 g d'iodure de triméthylsulfoxonium dans 150 ml de DMSO anhydre, on ajoute 11,1 g d'hydrure de sodium en dispersion à 80 % dans une huile minérale.

  Après fin de dégagement d'hydrogène (30 minutes environ) on ajoute, sans dépasser 35°C une solution de 54,6 g d'ester t. butylique préparé précédemment dans 60 ml de DMSO. Le mélange est agité à température ambiante durant 30 minutes puis à 55-60°C durant une heure et demie.

  La solution est alors refroidie à 25°C puis précipitée dans 1000 ml d'eau. Le mélange est extrait par 4 fois 100 ml d'éther, les phases éthérées réunies lavées par une solution saturée en NaCl puis séchées sur MgSO4. Après élimination des solvants par distillation sous vide, on obtient 47,7 g de trans 2-(2-thiényl)-1-cyclopropane carboxylate de t.butyle sous forme d'une huile jaune résiduelle qui est engagée telle quelle dans le stade qui suit .
- 3ème stade : Dans un réacteur anhydre, sous atmosphère d'azote, à une suspension de 6,9 g d'hydrure de lithium aluminium dans 75 ml de THF anhydre, on ajoute goutte à goutte sans dépasser 5°C, 20,4 g d'ester t.butylique obtenu au stade précédent en solution dans 75 ml de THF. Après retour à la température ambiante, le mélange est agité durant 2 heures, puis on ajoute goutte à goutte et avec précautions 16 ml d'eau, puis 16 ml d'une solution d'hydroxyde de sodium à 10 %. Après un nouvel ajout de 48 ml d'eau la suspension est filtrée, l'insoluble lavé à l'acétate d'éthyle puis les phases organiques réunies sont séchées sur sulfate de sodium. Les solvants sont éliminés par distillation sous vide. Le trans 1-hydroxyméthyl-2-(2-thiényl)-cyclopropane purifié est obtenu par chromatographie sur colonne de silice du résidu.

Poids : 16,4 g Rdt : 76 %

4ème stade : Sous atmosphère d'azote dans un réacteur anhydre on ajoute à 40,0 g de dichlorotriphénylphosphorane 15,5 g d'alcool obtenu au stade précédent en solution dans 75,0 ml de triéthylamine anhydre.

La suspension est maintenue sous agitation à 25°C durant 24 heures. Le mélange est précipité dans 500 ml d'eau et on ajoute 100 ml d'hexane. Le mélange est filtré, la phase aqueuse séparée et réextraite par 100 ml d'hexane. Les phases organiques réunies sont séchées sur MgSO4 puis les solvants éliminés par distillation sous vide. Le trans 1-chlorométhyl-2-(2- thiényl)-cyclopropane purifié sous forme d'huile incolore est obtenu par distillation sous vide de résidu.

Poids : 9,7 g Rdt : 56 % Eb = 65-70°C/40 Pa

A.6 - trans-1-chlorométhyl-2-(3-thiényl)-cyclopropane

(R5 = 3-thiényle ; Q = cyclopropane-1,2-diyle ; Z5 = Cl)

Le produit est obtenu à partir de 3-thiénylacrylate de méthyle (A.4 - 2ème stade) selon un mode opératoire semblable à celui décrit à la préparation du composé A.5 précédent.

- 1er stade : 3-thiénylacrylate de t.butyle
  Eb = 95-110°C/13 Pa
- 2ème stade : trans 2-(3-thiényl)-1-cyclopropane carboxylate de t.butyle - huile non purifiée
- 3ème stade : trans 1-hydroxyméthyl-2-(3-thiényl)-cyclopropane
  Eb = 88-120°C/20 Pa
- 4ème stade : trans 1-chlorométhyl-2-(3-thiényl)-cyclopropane
  Eb = 60-78°C/400 Pa

B- réactifs intermédiaires de formule (IX)

Mode opératoire général:

- stade 1 : préparation des amino nitriles (XII)

  Dans un réacteur on dissout dans 20 ml d'eau, 5,90 g (0,12 mole) de cyanure de sodium et 0,12 mole d'un sel hydrosoluble d'une amine R3-NH-(CH2)m-R4.

  A cette solution il est introduit en une heure et à une température comprise entre 30 et 40°C une solution de 0,10 mole d'aldéhyde R1-CHO dans 10 ml de méthanol. Le mélange est agité 4 heures à la température ambiante puis précipité dans 75 ml d'eau glacée puis extrait à l'éther.

  Les phases éthérées réunies sont lavées successivement à l'eau, par une solution à 25 % de bisulfite de sodium puis encore à l'eau.

  L'éther est ensuite évaporé et le produit brut résiduel est éventuellement purifié comme par exemple par distillation.
- stade 2 : préparation de l'intermédiaire (IX)

  Dans un réacteur protégé de l'humidité et sous atmosphère d'azote, 1,025 mole de n. butyllithium (solution 10M/ hexanes) est ajoutée goutte à goutte à 20°C dans une solution de 1,025 mole de diisopropylamine dans 1 litre de tetrahydrofurane anhydre.

  Le mélange est maintenu 15 minutes à 20°C. A - 72°C, il est introduit 1,0 mole d'amino-nitrile (XII) en solution dans 200 ml de THF, l'agitation est maintenue 1 h 30 à cette température puis on ajoute 1,025 mole du réactif d'alkylation (XIII) en solution dans 500 ml de THF. Après 20 minutes à - 72°C le mélange est agité 1 h à température ambiante.

  On ajoute ensuite 1,5 l de solution de NH4Cl à 10 % (p/v) et 750 ml de mélange hexanes-acétate d'éthyle 1-1 (v/v).

  La phase organique est séparée, la phase aqueuse réextraite par le mélange de solvants. Les phases organiques réunies sont lavées par extraction avec une solution saturée en chlorure de sodium, puis séchées sur MgSO4. Les solvants sont éliminés par distillation sous vide et sur bain-marie. Le produit brut huileux est généralement dans un état de pureté satisfaisant pour être engagé tel quel dans la suite des préparations des composés de l'invention.

B-1/ 1-cyano-1-(N-cyclopropylméthyl-N-méthyl)amino-1-(2-furyl)-4-phényl-but-3-ène.

(R1 = 2-furyle ; R3 =CH3 ; m = 1 ; R4 = (CH2)2CH ; R5 = C6H5 ; Q = -CH=CH-)de formule (IX)

La préparation de l'intermédiaire (XII) α-amino-N-cyclopropylméthyl-N-méthyl-(2-furyl)acétonitrile est réalisée à partir de 2-furaldéhyde, de N-méthyl N-cyclopropylméthylamine et de cyanure de sodium. Le produit huileux obtenu est purifié par distillation sous vide Eb = 82-92°C/15 Pa.

Selon le mode opératoire général décrit précédemment le produit est alkylé par le bromure de cinnamyle. Le composé huileux brut obtenu (Rdt = 100 %) est engagé tel quel dans la réaction avec le dérivé organo magnésien.

RMN : 0,10-0,15 (m, 5H) ; 2,10-2,45 (m, 2H) ; 2,55 (s, 3H) ; 2,90 (d, 2H) ; 5,70 (t, 1H) ; 6,35 (d, 1H) ; 6,85-6,95 (m, 1H) ; 7,10-7,45 (m,7H)

B-2/ <u>1-cyano-1-(N-cyclopropylméthyl-N-méthyl)amino-4-phényl-1-(2-thiényl)-but-3-ène.</u>

(R1 = 2-thiényle ; R3 =CH3 ; m = 1 ; R4 = (CH2)2CH ; R5 = C6H5 ; Q = -CH=CH-) de formule (IX)

L'intermédiaire (XII) est préparé à partir de 2-thiophènecarboxaldéhyde, de N-méthyl-N-cyclopropylméthylamine et de cyanure de sodium. Le dérivé α-amino-N-cyclopropylméthyl-N-méthyl-(2-thiényl) acétonitrile obtenu est utilisé sans purification.

Selon le mode opératoire général le composé est alkylé par le bromure de cinnamyle. Le produit huileux marron obtenu (Rdt = 100%) est engagé tel quel dans la réaction avec le dérivé organo magnésien.

RMN : 0,10-0,15 (m, 5H) ; 2,10-2,45 (m, 2H) ; 2,55 (s, 3H) ; 2,90 (d, 2H) ; 5,80 (t, 1H) ; 6,35 (d, 1H) ; 6,85-6,95) (m, 1H) ; 7,10-7,40 (m,7H)

B-3/ <u>1-cyano-1-(N-cyclopropylméthyl-N-méthyl)amino-4-phényl-1-(3-thiényl)-but-3-ène.</u>

(R1 = 3-thiényle ; R3 =CH3 ; m = 1 ; R4 = (CH2)2CH ; R5 = C6H5 ; Q = -CH=CH-) de formule (IX)

Le composé est préparé d'une façon identique au produit précédent B-2 à partir de 3-thiophène-carboxaldéhyde.

RMN : 0,0-1,10 (m,5H) ; 2,30 (m,2H) ; 2,50 (s,3H) ; 3,95 (q,2H) ; 5,80 (m,1H) ; 6,50 (d,1H) ; 7,00-7,50 (m,8H)

B-4/ <u>trans 1-[2-cyano-2-(2-furyl)-(N-cyclopropylméthyl-N-méthyl)aminoéthyl]-2-phényl-cyclopropane.</u>

(R1 = 2-furyle ; R3 =CH3 ; m = 1 ; R4 = (CH2)2CH ; R5 = C6H5 ; Q = cyclopropane-1,2-diyle) de formule (IX)

La préparation préliminaire de l'intermédiaire (XII) 1-(N-cyclopropylméthyl-N-méthyl)amino-(2-furyl) acétonitrile est réalisée à partir de 2-furaldéhyde, de N-méthyl-N-cyclopropylméthylamine comme décrit au mode opératoire général. Il en est de même pour l'alkylation par le trans-1-bromométhyl-2-phényl-cyclopropane dont la préparation est décrite en B-2. L'huile marron obtenue est utilisée sans purification dans la réaction qui suit avec le dérivé organo magnésien.

RMN : 0,10-0,90 (m, 8H); 1,30-1,80 (m, 1H); 2,20-2,30 (m,4H) ; 2,40 (s, 3H) ; 6,30 (m,1H) ; 6,50 (t, 1H) ; 7,10 (m, 5H) 7,40 (m, 1H)

B-5/ <u>trans 1-[2-cyano-2-(2-thiényl)-(N-cyclopropylméthyl-N-méthyl)aminoéthyl]-2-phényl-cyclopropane.</u>

(R1 = 2-thiényle ; R3 =CH3 ; m = 1 ; R4 = (CH2)2CH ; R5 = C6H5 ; Q = cyclopropane-1,2-diyle) de formule (IX)

A partir de l'intermédiaire décrit en B-2 et du dérivé bromé A-2 le produit marron huileux est utilisé sous forme non purifiée.

RMN : 0,10-1,10 (m, 10H) ; 2,10-2,40 (m, 3H) ; 2,60 (s, 3H) ; 6,75-6,90 (m, 1H) ; 7,05-7,40 (m, 7H)

B-6/ <u>trans 1-[2-cyano-2-(3-thiényl)-(N-cyclopropylméthyl-N-méthyl)aminoéthyl]-2-phényl-cyclopropane.</u>

(R1 = 3-thiényle ; R3 =CH3 ; m = 1 ; R4 = (CH2)2CH ; R5 = C6H5 ; Q = cyclopropane-1,2-diyle) de formule (IX)

A partir de l'intermédiaire (XII) décrit en B-3 et du dérivé bromé A-2 le produit huileux est utilisé sous forme non purifiée.

RMN : 0,30-1,90 (m, 9H) ; 2,20 (m, 4H) ; 2,45 (s, 3H) ; 6,80-7,50 (m, 8H)

B-7/ <u>1-cyano-1-(N-cyclopropylméthyl-N-méthyl)amino-1-(2-thiényl)-4-(3-thiényl)-but-3-ène.</u>

(R1 = 2-thiényle ; R3 = CH3 ; m = 1 ; R4 = (CH2)2CH ; R5 = 3-thiényl ; Q = -CH=CH-) de formule (IX)

L'aminonitrile intermédiaire (XII) est préparé à partir de 2-thiophènecarboxaldéhyde comme décrit pour

le composé B.2 puis condensé avec l'intermédiaire (XIII) selon le mode opératoire général. Le produit obtenu brut (Rdt : 100 %) est engagé tel quel dans la réaction avec le réactif organomagnésien.

B-8/ 1-cyano-1-(N-cyclopropylméthyl-N-méthyl)amino-1-(2-furyl)-4-(2-thiényl)-but-3-ène.

(R1 = 2-furyl ; R3 = CH3 ; m = 1 ; R4 = (CH2)2CH ; R5 = 2-thiényl ; Q = -CH=CH-) de formule (IX)

Le composé est préparé tel que décrit précédemment à partir de 2-furaldéhyde et d'intermédiaire (XIII) décrit en A.3. Le produit brut (Rdt : 100 %) est engagé tel quel avec le réactif organomagnésien.

B-9/ trans 1-[2-cyano-2-phényl-(N-cyclopropylméthyl-N-méthyl)aminoéthyl]-2-(2-thiényl)-cyclopropane.

(R1 = C6H5 ; R3 = CH3 ; m = 1 ; R4 = (CH2)2CH ; R5 = 2-thiényl ; Q = cyclopropane-1,2-diyle) de formule (IX)

Le composé est préparé tel que décrit précédemment à partir de benzaldéhyde et de l'intermédiaire (XIII) décrit en A.5. Le produit brut (Rdt : 100 %) est engagé tel quel dans la réaction avec le réactif organomagnésien.

RMN : 0,30-2,60 (m,6H) ; 2,25(t,2H) ; 2,50 (s,3H) ; 6,90 (m,3H) ; 7,37 (m,3H) ; 7,60 (m,2H)

B-10/trans 1-[2-cyano-2-(3-thiényl)-(N-cyclopropylméthyl-N-méthyl)aminoéthyl]-2-(3-thiényl)-cyclopropane.

(R1 = R5 = 3-thiényl ; R3 = CH3 ; m = 1 ; R4 = (CH2)2CH; Q = cyclopropane 1,2 diyle) de formule (IX).

Obtenu à partir de thiophènecarboxaldéhyde et de l'intermédiaire (XIII) décrit en A.6. Le composé brut (Rdt : 100 %) est utilisé sans purification.

N-CYCLOALKYLALKYLAMINES : EXEMPLES

Mode opératoire général :

Dans un réacteur protégé de l'humidité et sous atmosphère d'azote, on introduit 76,0 ml d'une solution éthérée de bromure d'éthyl magnésium 3M (227 mmol).

On ajoute ensuite à une température comprise entre 20 et 30°C, 50,4 mmol d'un amino nitrile (IX), en solution dans 65 ml de THF déshydraté sur tamis moléculaire.

Le mélange est agité 3 heures à une température d'environ 20°C puis introduit sans dépasser 10°C dans 260 ml d'une solution aqueuse saturée en chlorure d'ammonium.

La phase aqueuse est écartée et la phase organique extraite 3 fois par une solution 2N d'acide chlorhydrique. Les phases chlorhydriques réunies sont alcalinisées par une solution concentrée d'hydroxyde de sodium puis extraites à l'éther. Les phases éthérées sont réunies, lavées à l'eau puis déshydratées sur Na2SO4. Après élimination de l'éther par distillation, le produit est purifié par les techniques appropriées.

Ce mode opératoire est appliqué aux intermédiaires (IX) B-1 à B-10 avec le bromure d'éthyl magnésium pour obtenir les composés de l'invention de formule (I) des exemples 1 à 10.

Exemple 1. 4-(N-cyclopropylméthyl-N-méthyl)amino-4-(2-furyl)-1-phényl-hex-1-ène

(R1 = 2-furyle ; R2 = C2H5 ; R3 = CH3 ; m = 1 ; R4 = CH (CH2)2 ; R5 = C6H5 ; Q = -CH=CH-)
A partir de l'amino nitrile B-1
Huile jaune CCM = 0,55 ; S.B
IR : 3080, 3030, 2980, 2880, 2800, 1600, 1560, 1500, 1465, 1380, 1230, 1160, 1020, 980, 810, 740, 695 cm-1 RMN : 0,10-0,60 (m, 5H) ; 0,90 (t, 3H) ; 1,90 (q, 2H) ; 2,30 (d, 2H) ; 2,40 (s, 3H) ; 2,80-2,90 (m, 2H) ; 6,20-6,60 (m, 4H) ; 7,30-7,40 (m, 6H)
Anal. (C21H27NO) C, H, N, O.

Exemple 2. 4-(N-cyclopropylméthyl-N-méthyl)amino-1-phényl-4-(2-thiényl)-hex-1-ène

(R1 = 2-thiényle ; R2 = C2H5 ; R3 = CH3 ; m = 1 ; R4 = CH(CH2)2 ; R5 = C6H5 ; Q = -CH=CH-)
A partir de l'amino nitrile B-2
Huile jaune Eb = 160°C/35 Pa. CCM = 0,25 ; S.D
IR : 3070, 3020, 2960, 2930, 2895, 1665, 1640, 1595, 1570, 1490, 1450, 1230, 1030, 970, 825, 745, 690 cm-1 RMN : 0,05-0,55 (m, 5H) ; 1,90 (m, 5H) ; 2,30 (d, 2H) ; 2,50 (s, 3H) ; 2,90 (d, 2H) ; 6,10-6,60 (m, 2H) ; 7,00-7,10 (m, 2H) ; 7,20-7,30 (m, 6H)
Anal. (C21H27NS) C, H, N, S.

Exemple 3. 4-(N-cyclopropylméthyl-N-méthyl)amino-1-phényl-4-(3-thiényl)-hex-1-ène

(R1 = 3-thiényle ; R2 = C2H5 ; R3 = CH3 ; m = 1 ; R4 = CH (CH2)2 ; R5 = C6H5 ; Q = -CH=CH-)
A partir de l'amino nitrile B-3
Huile jaune Eb = 140°C/3,5 Pa. CCM = 0,45 ; S.A
IR : 3075, 3055, 3020, 2990, 2965, 2930, 2875, 2840, 2790, 1595, 1495, 1460, 1015, 980, 965, 845, 775, 740, 690, 665 cm-1
RMN : 0,10-0,55 (m, 4H) ; 0,70 (s, 1H) ; 0,75 (t, 3H) ; 1,85 (q, 2H) ; 2,25 (d, 2H) ; 2,40 (s, 3H) ; 6,00-6,50 (m, 2H) ; 7,00-7,40 (m, 8H)
Anal. (C21H27NS) C, H, N, S.

Exemple 4. trans 1-[2-(N-cyclopropylméthyl-N-méthyl)amino-2-(2-furyl)-butyl]-2-phényl-cyclopropane

(R1 = 2-furyle ; R2 = C2H5 ; R3 = CH3 ; m = 1 ; R4 = CH (CH2)2 ; R5 = C6H5 ; Q = cyclopropane-1,2-diyle)
A partir de l'amino nitrile B-4
Huile marron CCM = 0,45 ; S.B
IR : 3060, 2990, 2970, 2930, 2790, 1605, 1499, 1460, 1220, 1158, 1015, 940, 885, 830, 800, 730, 700 cm-1
RMN : 0,40-0,95 (m, 11H) ; 1,20-1,70 (m, 2H) ; 1,95 (m, 3H) ; 2,15 (m, 2H) ; 2,35 (s, 3H) ; 6,05 (m, 1H) ; 6,35 (m, 1H) ; 6,85-7,35 (m, 6H)
Anal. (C22H29NO) C, H, N, O.

Exemple 5. trans 1-[2-(N-cyclopropylméthyl-N-méthyl)amino-2-(2-thiényl)-butyl]-2-phényl-cyclopropane

(R1 = 2-thiényle ; R2 = C2H5 ; R3 = CH3 ; m = 1 ; R4 = CH (CH2)2 ; R5 = C6H5 ; Q = cyclopropane-1,2-diyle)
A partir de l'amino nitrile B-5
Huile marron CCM = 0,45 ; S.A
IR : 3060, 2980, 2960, 2940, 2860, 2790, 1610, 1500, 1465, 1240, 1020, 830, 750, 700 cm-1
RMN : 0,10 (m, 2H) ; 0,40-0,50 (m, 2H) ; 0,80-0,95 (m, 5H) ; 1,30 (s, 1H) ; 1,50-1,90 (m, 2H) ; 2,00-2,10 (m, 4H) ; 2,30 (d, 2H) ; 2,45 (s, 3H) ; 6,80-7,25 (m, 8H)
Anal. (C22H29NS) C, H, N, S.

Exemple 6. trans 1-[2-(N-cyclopropylméthyl-N-méthyl)amino-2-(3-thiényl)-butyl]-2-phényl-cyclopropane

(R1 = 3-thiényle ; R2 = C2H5 ; R3 = CH3 ; m = 1 ; R4 = CH (CH2)2 ; R5 = C6H5 ; Q = cyclopropane-1,2-diyle)
A partir de l'amino nitrile B-6
Huile marron CCM = 0,75 ; S.C
IR : 3070, 2990, 2965, 2930, 2875, 2840, 2785, 1604, 1497, 1460, 1015, 777, 750, 693 cm-1
RMN : 0,05-0,15 (m, 1H) ; 0,25-0,55 (m, 2H) ; 0,60-1,10 (m, 7H) ; 1,30-1,75 (m, 2H) ; 1,75-2,10 (m, 4H) ; 2,15-2,35 (m, 2H) ; 2,40 (s, 3H) ; 6,85-7,40 (m, 8H)
Anal. (C22H29NS) C, H, N, S.

Exemple 7. 4-(N-cyclopropylméthyl-N-méthyl)amino-4-(2-thiényl)-1-(3-thiényl)-hex-1-ène

(R1 = 2-thiényle ; R2 = C2H5 ; R3 = CH3 ; m = 1 ; R4 = (CH2)CH ; R5 = 3-thiényle ; Q = -CH=CH-)
A partir de l'amino nitrile B-7
Huile jaune Eb = 143-151°C/5 Pa. CCM = 0,50 ; S.A
IR (film) : 3076, 3050, 2968, 2942, 2908, 2848, 2820, 2768, 1448, 1237, 1222, 1074, 1008, 957, 852, 832, 793, 757, 687 cm-1
RMN : 0,10-0,20 (m, 2H) ; 0,30-0,70 (m, 2H) ; 0,70-1,10 (m, 1H) ; 0,85 (t, 3H) ; 1,90 (q, 2H) ; 2,30 (d, 2H) ; 2,45 (s, 3H) ; 2,80 (d, 2H) ; 6,10 (m, 1H) ; 6,52 (d, 1H) ; 6,80-7,50 (m, 6H)
Anal. (C19H25NS2) C, H, N, S.

Exemple 8. 4-(N-cyclopropylméthyl-N-méthyl)amino-4-(2-furyl)-1-(2-thiényl)-hex-1-ène

(R1 = 2-furyle ; R2 = C2H5 ; R3 = CH3 ; m = 1 ; R4 = (CH2)2CH ; R5 = 2-thiényle ; Q = -CH=CH-)

A partir de l'amino nitrile B-8
Huile jaune Eb = 125-145°C/10 Pa. CCM = 0,40 ; S.A
IR (film) : 3070, 2965, 2870, 2820, 2795, 1500, 1465, 1225, 1210, 1160, 1125, 1080, 1045, 1020, 990, 960, 860, 830, 805, 737, 695 cm-1
RMN : 0,10-0,20 (m, 2H) ; 0,20-0,60 (m, 2H) ; 0,60-1,10 (m, 4H) ; 1,88 (q, 2H) ; 2,26 (d, 2H) ; 2,37 (s, 3H) ; 2,50-3,20 (m, 2H) ; 5,80-6,20 (m, 2H) ; 6,20-6,40 (m,1H) 6,60 (d, 1H) ; 6,80-7,20 (m, 3H) ; 7,42 (s, 1H)
Anal. ($C_{19}H_{25}NOS$) C, H, N, O, S.

Exemple 9. trans 1-[2-(N-cyclopropylméthyl-N-méthyl) amino-2-phényl-butyl]-2-(2-thiényl)-cyclopropane

(R1 = C6H5 ; R2 = C2H5 ; R3 = CH3 ; m = 1 ; R4 = (CH2)2CH; R5 = 2-thiényle ; Q = cyclopropane 1,2-diyle)
A partir de l'amino nitrile B-9
Huile jaune Eb = 135-145°C/13 Pa. CCM = 0,50 ; S.B IR : 3062, 2962, 2786, 1596, 1490, 1442, 1018, 759, 697 cm-1
RMN : 0,30-0,60 (m, 2H) ; 0,60-1,20 (m, 8H) ; 1,60-2,20 (m, 6H) ; 2,35 (d, 2H) ; 2,45 (s, 3H) ; 6,50-6,70 (m, 1H) ; 6,80-6,90 (m, 1H) ; 6,90-7,10 (m, 1H) ; 7,10-7,60 (m, 5H)
Anal. ($C_{22}H_{29}NS$) C, H, N, S

Exemple 10. trans 1-[2-(N-cyclopropylméthyl-N-méthyl) amino-2-(3-thiényl)-butyl]-2-(3-thiényl)-cyclopropane

(R1 = R5 = 3-thiényle ; R2 = C2H5 ; R3 = CH3 ; m = 1 ; R4 = (CH2)2CH ; Q = cyclopropane-1,2-diyle)
A partir de l'amino nitrile B-10
Huile jaune Eb = 163-166°C/1 Pa. CCM = 0,25 ; S.A
IR (film) : 3060, 2960, 2780, 1680, 1460, 1020, 850, 780 cm-1
RMN : 0,30-0,60 (m, 2H) ; 0,60-1,10 (m, 7H) ; 1,50-2,40 (m, 9H) ; 2,37 (s, 3H) ; 6,70-6,90 (m, 2H) ; 7,00-7,40 (m, 4H)
Anal. ($C_{20}H_{27}NS_2$) C, H, N, S.

Les essais toxicologiques et pharmacologiques réalisés avec les composés des exemples 1 à 10 précédents mettent en évidence leur faible toxicité de même que, chez la souris, la capacité d'inhiber les convulsions induites par la picrotoxine.

Cette propriété permet d'envisager une utilité des produits de l'invention comme psychotropes dans le traitement des affections neuropsychiques.

Par ailleurs, les affinités de liaison "in vitro" aux récepteurs opioïdes mu, delta, kappa ont été étudiées, de même que celles aux récepteurs sigma.

Les résultats des études effectuées montrent pour les composés de l'invention une affinité de liaison particulière pour les récepteurs sigma en relation avec leur activité anti-psychotique et, plus manifestement, lorsque dans les composés R1 est un radical 2-thiènyle et R5 est un radical phényle.

De même, "in vivo" chez le rat, les cycloalkylalkylamines (I) inhibent au niveau gastro-duodénal les ulcères provoqués par administration de cystéamine. Cette activité est imputée également à l'affinité particulière des composés aux récepteurs sigma locaux.

L'ensemble de ces propriétés, concrétisé par les études effectuées et leurs résultats présentés dans le présent mémoire rendent les composés selon l'invention utiles à la prévention et au traitement des asthénies et des désordres d'ordre neurologique et/ou psychique de même qu'au traitement de divers dysfonctionnements du tractus gastro-intestinal.

Les études démonstratives des propriétés des produits de l'invention et les résultats obtenus sont rapportés ci-après :

a) la toxicité des produits de l'invention est recherchée chez la souris par la détermination approchée de leur DL 50, qui est la dose léthale provoquant 50 % de morts chez les animaux dans les conditions de l'expérience. L'étude est réalisée sur des lots de quatre souris "Swiss" mâles d'un poids de 20 g environ mises à jeun la veille de l'étude.

Chaque détermination est effectuée avec quatre doses correspondant respectivement à une administration par voie orale de 100, 300, 600 et 1000 mg de produit, exprimées sous forme de base, par kg d'animal.

Il est constaté par cette étude que les produits de l'invention ont une toxicité aigüe correspondant à une DL50 supérieure ou égale à 1.000 mg/kg. Exceptionnellement, cette toxicité peut-être d'environ 600 mg/kg.

b) Les propriétés psychotropes des composés ont été déterminées par la protection des convulsions induites par la picrotoxine chez la souris, qui a été réalisée selon une méthode dérivée de celle de Krall et coll., "Epilepsia", 1978, 19, p.409-428.

L'administration de picrotoxine provoque chez l'animal une crise convulsive caractérisée par un syndrome d'extension myoclonique suivi de l'extension des membres conduisant à la mort de l'animal. Certaines substances, notamment celles actives sur le complexe GABA/benzodiazépines/Cl-ionophore permettent de protéger les animaux de cette crise convulsive.

Pratiquement l'étude est réalisée sur des lots de 10 souris "Swiss" mâles, d'un poids de 20 g environ auxquelles on administre le produit à étudier en solution aqueuse soit par voie intrapéritonéale (i.p.) soit par voie orale (p.o).

On pratique ensuite une injection par voie intrapéritonéale d'une solution de picrotoxine à raison de 24 mg/kg sous un volume de 0,2 ml par animal, soit 30 minutes après l'administration du produit par voie intrapéritonéale, soit 60 minutes après l'administration du produit par voie orale, la dose de produit ainsi injectée provoquant une crise clonique qui conduit à la mort des animaux non traités. Dans les conditions du test, on observe chez les animaux traités la suppression de la phase tonique d'extension.

Les résultats sont exprimés :

- soit en pourcentage d'animaux protégés de cette phase sous l'action de 50 mg/kg du composé à l'étude administré par voie i.p. ou 100 mg/kg par voie p.o.,
- soit en DE50 pour chacune de ces voies, qui est la dose efficace de composé à l'essai exprimée en mg/kg, protégeant 50 % des animaux de cette phase d'extension, la valeur significative des résultats étant généralement indiquée de la façon suivante :

* Résultat significatif à p. < 0,05

** Résultat significatif à p. < 0,01

Les résultats de l'étude sont reportés pour les produits de l'invention dans le tableau 1 qui suit. Ils montrent pour les deux voies d'administration l'activité protectrice des composés de l'invention étudiés.

Tableau 1 : Inhibition des convulsions induites par la picrotoxine

| Exemple | i.p. : % prot. ou DE50 | p.o. : % prot. ou DE50 |
|---|---|---|
| 1 | N.T. | 60 % ** |
| 2 | 50 % * | N.T. |
| 4 | 50 % * | N.T. |
| 5 | 50 % * | 50 % * |
| 6 | 50 % * | 50 % * |
| 9 | N.T. | DE 50 = 90 mg |
| 10 | 50 % * | DE 50 = 74 mg |

N.T. Non testé

c) l'étude "in vitro" de l'affinité des composés aux récepteurs sigma est réalisée selon la technique décrite par Largent B.L. et coll. dans J. Pharmacol. Exp. Thér. 238, 1986, p. 739-748 dont le principe est de mettre en compétition les affinités respectives du produit à étudier et celle du (+) [3H] SKF 10,047 qui est le ligand radioactif caractéristique des récepteurs sigma des membranes de cerveau de cobaye que l'on utilise dans cette étude.

L'essai est réalisé en faisant incuber des solutions de concentrations appropriées des produits avec des prélèvements standards de membranes puis à déterminer après filtration, la radioactivité résiduelle de la solution.

Les résultats sont traités de façon à calculer la CI50 du produit à l'étude qui est, dans ce cas, la concentration nanomolaire de solution capable d'inhiber 50 % des liaisons du ligand radioactif aux récepteurs sigma des membranes utilisées.

Les résultats sont présentés au tableau 2 qui suit et, à titre de référence, il est aussi présenté celui obtenu avec la Ditolylguanidine (DTG) qui est un réactif pharmacologique de référence considérée comme un ligand sélectif et de grande affinité aux récepteurs sigma (Stephen G. Holtzman. J. Pharm. Exp. Ther. Vol. 248, n° 3, 1989, p. 1054- 1062).

Tableau 2 : Affinité de liaison des composés de l'invention aux récepteurs sigma

| Composés à l'essai Exemple | CI50 (nmol) |
|---|---|
| 2 | 95 |
| 5 | 29 |
| DTG | 103 |

Ces résultats montrent que les produits mis à l'essai ont une affinité aux récepteurs sigma du même ordre de grandeur à 3 fois supérieure à celle de la DTG.

Ces affinités, associées aux faibles toxicités des cycloalkylalkylamines (I) sont démonstratives de leur intérêt.

La spécificité d'affinité des composés pour les récepteurs sigma est montrée par l'étude comparée de l'affinité des produits aux récepteurs opiacés mu, delta et kappa, ainsi qu'aux récepteurs de la phencyclidine (PCP) qui sont des récepteurs reconnus être impliqués comme médiateurs dans l'effet de drogues psychomimétiques (Eric J. Simon - "Opiates receptor binding in Drug Research" p. 183-199 dans "Receptor Binding in Drug Research" - Ed. Robert A. O'Brien-Marcel Dekker - 1986 et également Brian L. Largent et coll. dans Eur. J. of Pharmacol. 155 (1988) p. 345-347).

L'étude "in vitro" de l'affinité des composés des produits des exemples 2 et 5 de l'invention aux trois récepteurs opiacés est réalisée selon la technique décrite par F. Roman et coll. dans J. Pharm. Pharmacol. 1987, 39, p. 404-407 et l'étude de l'affinité aux récepteurs de la PCP est réalisée selon la technique décrite par Vignon J. et coll. dans "Brain Res." 1983 ; 280, p. 194-7 et 1986 ; 378, p. 133-41.

Les résultats présentés au tableau 3 sont, pour chaque récepteur étudié, exprimés en CI50 qui sont les concentrations nanomolaires des produits en solutions capables d'inhiber 50 % des liaisons du ligand radioactif spécifique lié au récepteur considéré.

**Tableau 3 : Affinité des produits de l'invention aux récepteurs sigma comparée à leurs affinités aux récepteurs mu, delta, kappa et PCP**

| Composés à l'essai: Exemple | Rec. mu | Rec. delta | Rec. Kappa | Rec. Sigma | Rec. PCP |
|---|---|---|---|---|---|
| 2 | 1542 | 7387 | 1196 | 95 | 6280 |
| 5 | 3024 | 57043 | 9095 | 29 | 4226 |
| DTG | 3970 | 33200 | 6490 | 103 | 6750 |

La spécificité d'affinité sigma est manifeste pour les composés préférés de l'invention.

En attribuant arbitrairement et pour comparaison la valeur 1 à la CI50 d'affinité sigma, on calcule les CI50 aux récepteurs mu, delta, kappa et PCP pour le composé de l'exemple 5 dont l'affinité sigma est la plus intense. On compare ces valeurs à celles obtenues avec le DTG présenté comme composé de référence.

| Composés à l'essai: Exemple | Rec. mu | Rec. delta | Rec. Kappa | Rec. Sigma | Rec. PCP |
|---|---|---|---|---|---|
| 5 | 104 | 1967 | 314 | 1 | 145 |
| DTG | 38 | 322 | 63 | 1 | 65 |

Ce mode d'expression montre que, dans les conditions opératoires décrites, l'un des composés préférés de l'invention possède une affinité pour les récepteurs sigma au moins 100 fois supérieure à celle déterminée pour les récepteurs mu, kappa et ceux de la PCP, et, dans le cas du récepteur delta, une affinité environ 2000 fois supérieure.

Comparé aux mêmes indices calculés pour le DTG, le composé de l'invention préparé à l'exemple 5 se montre au moins deux fois supérieur dans cette sélectivité d'affinité que cette référence.

d) L'activité des composés de l'invention sur le tractus gastrointestinal a été montrée chez le rat par leur aptitude à inhiber les ulcères gastroduodénaux provoqués par administration de cystéamine.

Cette propriété a été mise en évidence chez le rat selon une technique décrite par Robert et coll., dans "Digestion", 1974, 11, p. 199-211.

Dans les essais, des lots de 6 rats Wistar femelle d'un poids moyen de 200 g reçoivent une injection sous cutanée de chlorhydrate de cystéamine à raison de 400 mg/kg. Les produits à tester sont administrés par voie orale ou par voie sous cutanée respectivement 1 h ou 30 minutes avant l'agent ulcérogène

Dix-huit heures après, les rats sont sacrifiés par élongation, leur estomac et duodénum prélevés, rincés avec du soluté physiologique et épinglés sur un carton. La présence d'ulcères de la zone antro-pyloro duodénale est recherchée et leur surface, exprimée en mm2, est évaluée en multipliant les deux axes perpendiculaires principaux de la lésion. L'analyse statistique des résultats est effectuée à l'aide du test de Student pour les surfaces ulcérées, comparativement à un groupe contrôle ne recevant que l'excipient.

Les résultats présentés au tableau 4, sont exprimés en DE50 de scores d'ulcération qui sont les doses efficaces exprimées en mg/kg de produit qui provoquent l'inhibition de 50 % des ulcérations provoquées par la cystéamine.

**Tableau 4 : Activité inhibitrice sur les ulcères gastro-duodénaux provoqués par la cystéamine**

| Produit à l'essai Exemple | DE50 - Scores d'ulcérations mg/kg |
|---|---|
| 2 | 48,2 |
| 5 | 45,7 |

Ces propriétés pharmacologiques, associées à la faible toxicité des composés de l'invention permettent d'envisager leur utilité, sous forme de médicaments, aux traitements préventifs et curatifs d'affections d'ordre neurologique et/ou psychique en général, comme, par exemples, les états dépressifs, les troubles de la mémoire et/ou du comportement, la schizophrénie, la maladie d'Alzheimer, la maladie de Parkinson et la démence sénile.

Egalement les cycloalkylalkylamines (I) de l'invention sont appropriées au traitement des dysfonctionnements du tractus gastrointestinal en général comme par exemples les troubles du péristaltisme, de la motricité, les phénomènes de reflux gastro-oesophagiens et gastroduodénaux ainsi que les ulcérations gastriques et/ou gastroduodénales.

De façon habituelle les doses unitaires utiles sont comprises entre 1 et 500 mg et plus particulièrement entre 5 et 200 mg de produit selon la nature et la gravité de l'affection a traiter. Les doses thérapeutiques journalières peuvent être réparties en plusieurs prises et sont comprises entre 0,5 et 1500 mg de produit par jour. De façon générale une posologie journalière de 5 à 500 mg de produit par jour répartis en deux à quatre prises est satisfaisante.

L'administration des produits de l'invention aux patients à traiter est réalisée sous la forme de médicaments de nature adaptée à l'affection à soigner. Selon les cas les préparations médicamenteuses seront, comme exemples non limitatifs, des comprimés, dragées, capsules, poudres, solutions, suspensions, gels ou suppositoires. Ces formes pharmaceutiques sont préparées à partir des produits sous forme de base ou de leurs sels et selon des méthodes couramment pratiquées dans cette industrie.

Généralement dans les formes médicamenteuses de nature solide, le principe actif représente de 5 à 90 % en poids du total de la forme terminée alors que les excipients pharmaceutiquement acceptables représentent de 95 à 10 %. Pour les formes liquides, ou pouvant être considérées comme telles, la quantité de principe actif est comprise entre 0,1 et 10 % en poids de la forme terminée alors que les excipients pharmaceutiquement acceptables peuvent représenter de 99,9 à 90 % en poids de cette forme.

A titre d'illustration il est décrit la formule et la préparation de comprimés avec le composé de l'exemple 5

- Formule

| | |
|---|---|
| Principe actif (composé de l'ex. 5) | 10,0 à 50,0 mg |
| Polyvinylpyrrolidone | 20,0 mg |
| Carboxyméthylamidon | 8,0 mg |
| Stéarate de magnésium | 2,0 mg |
| Silice colloïdale | 0,4 mg |
| Lactose en quantité suffisante pour | 200,0 mg |

- Préparation

  Le principe actif en solution hydro alcoolique est mélangé au lactose puis granulé avec la polyvinyl pyrrolidone également en solution. Les grains sont séchés et tamisés sur une grille d'ouverture de 1 mm. Le carboxyméthylamidon est mélangé à la silice colloïdale puis ajouté aux granulés. On mélange ensuite intimement avec le stéarate de magnésium puis comprime à raison de 200,0 mg par comprimé.

**Revendications**

**Revendications pour les Etats contractants suivants : AT, BE, CH, DE, DK, FR, GB, IT, LI, LU, NL, SE**

**1.** N-cycloalkylalkylamines de formule générale (I) :

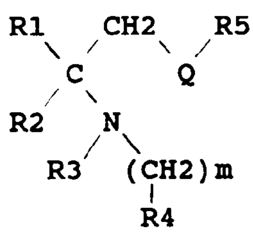

(I)

Dans laquelle :

R1 est un radical aromatique thiényle ou furyle ou un radical phényle sous réserve que Q représente un groupe cyclopropane-1,2-diyle

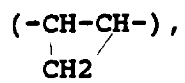

R2 est un alkyle de $C_1$ à $C_5$,

R3 est un hydrogène ou alkyle de $C_1$ à $C_5$,

m vaut 1 ou 2,

R4 est un radical cycloalkyle -CH(CH2)n, dans lequel n est un nombre entier de 2 à 5,

R5 est un radical phényle pouvant être éventuellement mono, di ou trisubstitué par des halogènes ou par des radicaux alkyle de $C_1$ à $C_5$ ou par des radicaux alkoxy jusqu'à $C_3$ éventuellement substitués par des atomes d'halogène, identiques ou différents, ou représente un radical thiényle,

Q représente un groupe éthylène-1-2-diyle (-CH=CH-) ou un groupe cyclopropane-1,2-diyle

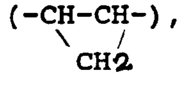

et leurs sels d'additions avec les acides.

2. N-cycloalkylalkylamines selon la revendication 1, caractérisées en ce que R1 est thiényle.

3. N-cycloalkylalkylamines selon la revendication 1 ou 2, caractérisées en ce que R5 est phényle.

4. N-cycloalkylalkylamines selon l'une des revendications 1 à 3, caractérisées en ce que R2 est éthyle.

5. N-cycloalkylalkylamines selon l'une des revendications 1 à 4, caractérisées en ce que R3 est méthyle.

6. N-cycloalkylalkylamines selon l'une des revendications 1 à 5, caractérisées en ce que m vaut 1 et R4 est cyclopropyle.

7. Le trans 1-[2-(N-cyclopropylméthyl-N-méthyl) amino-2-(2-thiényl)-butyl]-2-phényl-cyclopropane et ses sels d'additions avec des acides.

8. Le 4-(N-cyclopropylméthyl-N-méthyl)-amino-1-phényl-4-(2-thiényl)-hex-1-ène, et ses sels d'additions avec les acides.

9. Procédé de préparation des N-cycloalkylalkylamines (I) telles que définies à l'une des revendications 1 à 8, caractérisé en ce qu'il consiste
   - pour préparer un composé (II) de formule générale (I) et dans laquelle R3 est l'hydrogène,
     i) à réduire un N-carboxamide intermédiaire (IV)

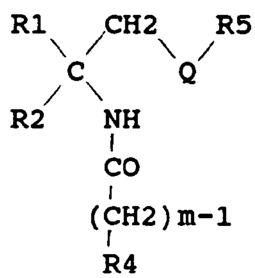

IV

par un hydrure métallique (Hm) de formule :

M1(t)M2H(2)Rx(s) (Hm)

dans laquelle :

M1 représente un atome alcalin comme le lithium ou le sodium et dont l'indice représentatif (t) a pour valeurs 0 ou 1,

M2 est un élément du groupe III de la classification périodique des éléments et de préférence le bore ou l'aluminium, (r) est l'indice représentatif du nombre d'atomes d'hydrogène de l'hydrure et a pour valeur 1, 2, 3 ou 4,

Rx est un groupe carbonitrile, un radical alkyle de $C_1$ à $C_5$ ou alkoxy jusqu'à $C_3$ éventuellement substitué par des atomes d'halogène dont l'indice représentatif (s) a pour valeur 0, 1, 2 ou 3,

les indices (t), (r) et (s) ci-dessus vérifiant la relation : (r) + (s) - (t) = 3, cette réduction étant effectuée de préférence par un hydrure (Hm.2) dans lequel M2 est l'aluminium ou encore le bore lorsque (r) vaut 3 et (s) et (t) ont pour valeur 0,

ii) à alkyler une amine (V) de formule

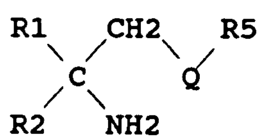

(V)

par un halogénure d'alkyle R4-(CH2)m-Z2 dans lequel Z2 est un halogène tel que le chlore, le brome ou l'iode,

- pour préparer une N-cycloalkylalkylamine (III) de formule générale (I) dans laquelle R3 est alkyle

en $C_1$ à $C_5$

i) à procéder à une alkylation réductrice d'un composé (II) de l'invention qui consiste à faire réagir ce composé (II) avec un aldéhyde R6-CHO dans lequel R6 est l'homologue carboné immédiatement inférieur au rédical R3 (R3 = CH2-R6), en présence d'un agent réducteur comme un hydrure métallique ou organométallique (Hm.3) de formule (Hm) définie ci-dessus et dans laquelle M2 est le bore et, d'une façon préférée Rx est un groupe carbonitrile, ou

ii) à réduire par un hydrure métallique (Hm.2) précédemment défini un N-carboxamide intermédiaire (VIII)

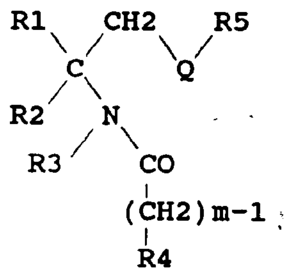

(VIII)

iii) ou à faire réagir un réactif organomagnésien R2MgZ3 dans lequel Z3 est un halogène comme le chlore, le brome, ou l'iode sur un intermédiaire aminonitrile (IX)

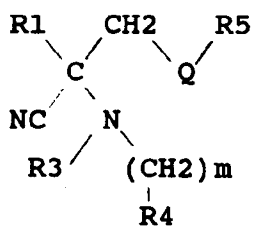

(IX)

iiii) ou encore à alkyler par un halogénure d'alkyle R4-(CH2)m-Z2 dans lequel Z2 est le chlore, le brome, ou l'iode une amide (VII) de formule

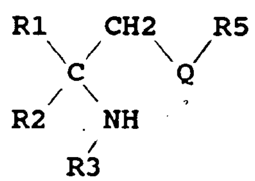

(VII)

**10.** Médicament, caractérisé en ce qu'il comprend une N-cycloalkylalkylamine de formule (I) telle que définie à l'une des revendications 1 à 8.

**11.** Utilisation d'une N-cylcloalkylalkylamine de formule (I) telle que définie à l'une des revendications 1 à 8, pour l'élaboration d'un médicament destiné aux traitements d'affections neurologiques et/ou psychiques.

**12.** Utilisation d'une N-cycloalkylalkylamine de formule (I) telle que définie à l'une des revendications 1 à 8, pour l'élaboration d'un médicament destiné au traitement des dysfonctionnements du tractus gastrointestinal.

**13.** Utilisation d'une N-cycloalkylalkylamine de formule (I) telle que définie à l'une des revendications 1 à 8, pour l'élaboration d'un médicament destiné au traitement des ulcérations gastriques et/ou gastroduodénales.

**Revendications pour l'Etat contractant suivant : ES**

**1.** Procédé de préparation de N-cycloalkylalkylamines de formule générale (I) :

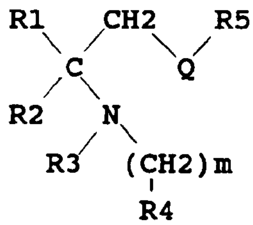

(I)

dans laquelle :

R1 est un radical aromatique thiényle ou furyle ou un radical phényle sous réserve que Q représente un groupe cyclopropane-1,2-diyle

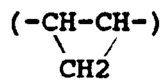,

R2 est un alkyle de $C_1$ à $C_5$

R3 est un hydrogène ou alkyle de $C_1$ à $C_5$

m vaut 1 ou 2,

R4 est un radical cycloalkyle -CH(CH2)n, dans lequel n est un nombre entier de 2 à 5,

R5 est un radical phényle pouvant être éventuellement mono, di ou trisubstitué par des halogènes ou par des radicaux alkyle de $C_1$ à $C_5$ ou par des radicaux alkoxy jusqu'à $C_3$ éventuellement substitués par des atomes d'halogène, identiques ou différents,
représente un radical thiényle,

Q représente un groupe éthylène-1-2-diyle (-CH=CH-) ou un groupe cyclopropane-1,2-diyle

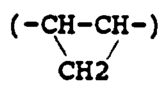,

et leurs sels d'additions avec des acides,
caractérisé en ce qu'il consiste

- pour préparer un composé (II) de formule générale (I) et dans laquelle R3 est l'hydrogène,
  i) à réduire un N-carboxamide intermédiaire (IV)

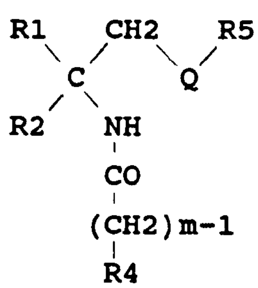

(IV)

par un hydrure métallique (Hm) de formule :

M1(t)M2H(2)Rx(s) (Hm)

dans laquelle :
M1 représente un atome alcalin comme le lithium ou le sodium et dont l'indice représentatif (t) a pour valeurs 0 ou 1,
M2 est un élément du groupe III de la classification périodique des éléments et de préférence le bore ou l'aluminium, (r) est l'indice représentatif du nombre d'atomes d'hydrogène de l'hydrure et a pour valeur 1, 2, 3 ou 4,
Rx est un groupe carbonitrile, un radical alkyle de $C_1$ à $C_5$ ou alkoxy jusqu'à $C_3$ éventuellement substitué par des atomes d'halogène dont l'indice représentatif (s) a pour valeur 0, 1, 2 ou 3,
les indices (t), (r) et (s) ci-dessus vérifiant la relation : (r) + (s) - (t) = 3. Cette réduction étant effectuée de préférence par un hydrure (Hm.2) dans lequel M2 est l'aluminium ou encore le bore lorsque (r) vaut 3 et (s) et (t) ont pour valeur 0,

ii) à alkyler une amine (V) de formule

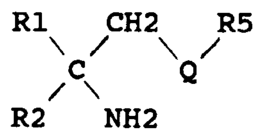

(V)

par un halogénure d'alkyle R4-(CH2)m-Z2 dans lequel Z2 est un halogène tel que le chlore, le brome ou l'iode,

- pour préparer une N-cycloalkylalkylamine (III) de formule générale (I) dans laquelle R3 est alkyle de $C_1$ à $C_5$

i) à procéder à une alkylation réductrice d'un composé (II) de l'invention qui consiste à faire réagir ce composé (II) avec un aldéhyde R6-CHO dans lequel R6 est l'homologue carboné immédiatement inférieur au radical R3 (R3 = CH2-R6), en présence d'un agent réducteur comme un hydrure métallique ou organométallique (Hm.3) de formule (Hm) définie ci-dessus et dans laquelle M2 est le bore et, d'une façon préférée Rx est un groupe carbonitrile, ou

ii) à réduire par un hydrure métallique (Hm.2) précédemment défini un N-carboxamide intermédiaire (VIII)

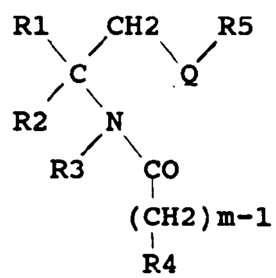

(VIII)

iii) ou à faire réagir un réactif organomagnésien R2MgZ3 dans lequel Z3 est un halogène comme le chlore, le brome, ou l'iode sur un intermédiaire aminonitrile (IX)

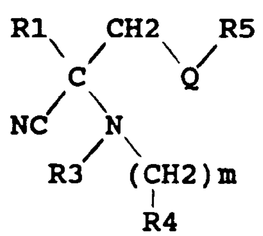

(IX)

iiii) ou encore à alkyler par un halogénure d'alkyle R4-(CH2)m-Z2 dans lequel Z2 est le chlore, le brome, ou l'iode une amide (VII) de formule

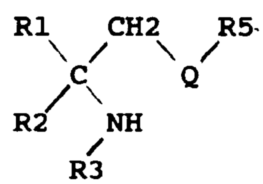

(VII)

2. Procédé suivant la revendication 1, caractérisé en ce que R1 est thiényle.

3. Procédé suivant la revendication 1 ou 2, caractérisé en ce que R5 est phényle.

4. Procédé suivant l'une des revendications 1 à 3, caractérisé en ce que R2 est éthyle.

5. Procédé suivant l'une des revendications 1 à 4, caractérisé en ce que R3 est méthyle.

6. Procédé suivant l'une des revendications 1 à 5, caractérisé en ce que m vaut 1 et R4 est cyclopropyle.

7. Procédé suivant la revendication 1, caractérisé en ce que R1 est 2-thiényle, R2 est éthyle, R3 est méthyle, m est égal à 1, R4 est cyclopropyle, R5 est phényle et Q est cyclopropane-1,2-diyle.

8. Procédé suivant la revendication 1, caractérisé en ce que R1 est 2-thiényle, R2 est éthyle, R3 est méthyle, m est égal à 1, R4 est cyclopropyle, R5 est phényle et Q est éthylène-1,2-diyle.

9. Procédé de préparation d'un médicament, caractérisé en ce qu'il consiste à mélanger une N-cycloalkyalkylamine de formule (I) telle que définie à l'une des revendications 1 à 8, à un excipient pharmaceutiquement acceptable.

10. Utilisation d'une N-cylcloalkylalkylamine de formule (I) telle que définie à l'une des revendications 1 à 8, pour l'élaboration d'un médicament destiné aux traitements d'affections neurologiques et/ou psychiques.

11. Utilisation d'une N-cycloalkylalkylamine de formule (I) telle que définie à l'une des revendications 1 à 8, pour l'élaboration d'un médicament destiné au traitement des dysfonctionnements du tractus gastrointestinal.

12. Utilisation d'une N-cycloalkylalkylamine de formule (I) telle que définie à l'une des revendications 1 à 8, pour l'élaboration d'un médicament destiné au traitement des ulcérations gastriques et/ou gastroduodénales.

**Revendications pour l'Etat contractant suivant : GR**

1. N-cycloalkylalkylamines de formule générale (I) :

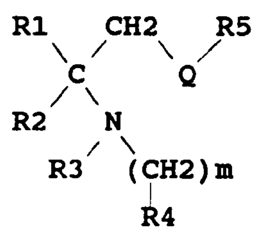

(I)

Dans laquelle :

R1 est un radical aromatique thiényle ou furyle ou un radical phényle sous réserve que Q représente un groupe cyclopropane-1,2-diyle

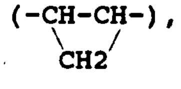

R2 est un alkyle de $C_1$ à $C_5$

R3 est un hydrogène ou alkyle de $C_1$ à $C_5$

m vaut 1 ou 2,

R4 est un radical cycloalkyle -CH(CH2)n, dans lequel n est un nombre entier de 2 à 5,

R5 est un radical phényle pouvant être éventuellement mono, di ou trisubstitué par des halogènes ou par des radicaux alkyle de $C_1$ à $C_5$ ou alkoxy jusqu'à $C_3$ éventuellement substitué par des atomes d'halogène, identiques ou différents, représente un radical thiényle,

Q représente un groupe éthylène-1-2-diyle (-CH=CH-) ou un groupe cyclopropane-1,2-diyle

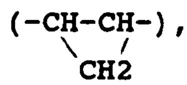

et leurs sels d'additions avec des acides.

2. N-cycloalkylalkylamines selon la revendication 1, caractérisées en ce que R1 est thiényle.

3. N-cycloalkylalkylamines selon la revendication 1 ou 2, caractérisées en ce que R5 est phényle.

4. N-cycloalkylalkylamines selon l'une des revendications 1 à 3, caractérisées en ce que R2 est éthyle.

5. N-cycloalkylalkylamines selon l'une des revendisations 1 à 4, caractérisées en ce que R3 est méthyle.

6. N-cycloalkylalkylamines selon l'une des revendications 1 à 5, caractérisées en ce que m vaut 1 et R4 est cyclopropyle.

7. Le trans 1-[2-(N-cyclopropylméthyl-N-méthyl) amino-2-(2-thiényl)-butyl]-2-phényl-cyclopropane et ses sels d'additions avec des acides.

8. Le 4-(N-cyclopropylméthyl-N-méthyl)-amino-1-phényl-4-(2-thiényl)-hex-1-ene, et ses sels d'additions avec les acides.

9. Procédé de préparation des N-cycloalkylalkylamines (I) telles que définies à l'une des revendications 1 à 8, caractérisé en ce qu'il consiste
   - pour préparer un composé (II) de formule générale (I) et dans laquelle R3 est l'hydrogène,
     i) à réduire un N-carboxamide intermédiaire (IV)

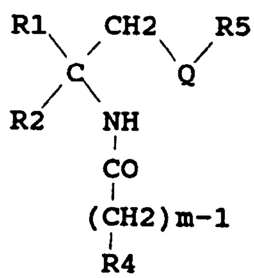

(IV)

par un hydrure métallique (Hm) de formule :

M1(t)M2H(2)Rx(s) (Hm)

dans laquelle :
M1 représente un atome alcalin comme le lithium ou le sodium et dont l'indice représentatif (t) a pour valeurs 0 ou 1,
M2 est un élément du groupe III de la classification périodique des éléments et de préférence le bore ou l'aluminium, (r) est l'indice représentatif du nombre d'atomes d'hydrogène de l'hydrure et a pour valeur 1, 2, 3 ou 4,
Rx est un groupe carbonitrile, un radical alkyle de $C_1$ à $C_5$ ou alkoxy jusqu'à $C_3$ éventuellement substitué par des atomes d'halogène dont l'indice représentatif (s) a pour valeur 0, 1, 2 ou 3,
les indices (t), (r) et (s) ci-dessus vérifiant la relation : (r) + (s) - (t) = 3, cette réduction étant effectuée de préférence par un hydrure (Hm.2) dans lequel M2 est l'aluminium ou encore le bore lorsque (r) vaut 3 et (s) et (t) ont pour valeur 0,
ii) à alkyler une amine (V) de formule

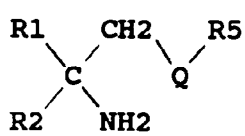

(V)

par un halogénure d'alkyle R4-(CH2)m-Z2 dans lequel Z2 est un halogène tel que le chlore, le brome ou l'iode,

- pour préparer une N-cycloalkylalkylamine (III) de formule générale (I) dans laquelle R3 est alkyle de $C_1$ à $C_5$

  i) à procéder à une alkylation réductrice d'un composé (II) de l'invention qui consiste à faire réagir ce composé (II) avec un aldéhyde R6-CHO dans lequel R6 est l'homologue carboné immédiatement inférieur au rédical R3 (R3 = CH2-R6), en présence d'un agent réducteur comme un hydrure métallique ou organométallique (Hm.3) de formule (Hm) définie ci-dessus et dans laquelle M2 est le bore et, d'une façon préférée Rx est un groupe carbonitrile, ou

  ii) à réduire par un hydrure métallique (Hm.2) précédemment défini un N-carboxamide intermédiaire (VIII)

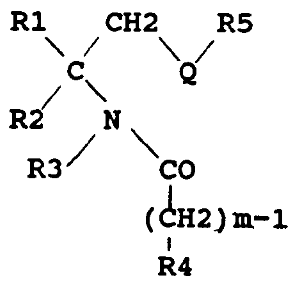

(VIII)

  iii) ou à faire réagir un réactif organomagnésien R2MgZ3 dans lequel Z3 est un halogène comme le chlore, le brome, ou l'iode sur un intermédiaire aminonitrile (IX)

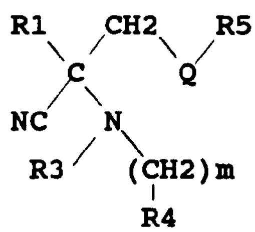

(IX)

  iiii) ou encore à alkyler par un halogénure d'alkyle R4-(CH2)m-Z2 dans lequel Z2 est le chlore, le brome, ou l'iode une amide (VII) de formule

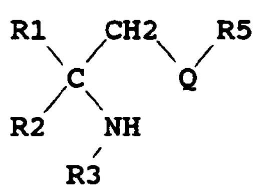

(VII)

**10.** Procédé de préparation d'un médicament caractérisé en ce qu'il consiste à mélanger une N-cycloalkyalkylamine de formule (I) telle que définie à l'une des revendications 1 à 8, à un excipient pharmaceutiquement acceptable.

**11.** Utilisation d'une N-cylcloalkylalkylamine de formule (I) telle que définie à l'une des revendications 1 à 8, pour l'élaboration d'un médicament destiné aux traitements d'affections neurologiques et/ou psychiques.

**12.** Utilisation d'une N-cycloalkylalkylamine de formule (I) telle que définie à l'une des revendications 1 à 8, pour l'élaboration d'un médicament destiné au traitement des dysfonctionnements du tractus gastrointestinal.

**13.** Utilisation d'une N-cycloalkylalkylamine de formule (I) telle que définie à l'une des revendications 1 à 8,

pour l'élaboration d'un médicament destiné au traitement des ulcérations gastriques et/ou gastroduodénales.

## Patentansprüche

### Patentansprüche für folgende Vertragsstaaten : AT, BE, CH, DE, DK, FR, GB, IT, LI, LU, NL, SE

1. N-Cycloalkylalkylamine der allgemeinen Formel (I)

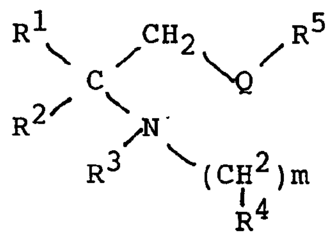

in der

$R^1$ ein aromatischer Thienyl- oder Furylrest oder ein Phenylrest ist, sofern Q eine Cyclopropan-1,2-diyl-Gruppe

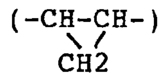

ist,

$R^2$ ein Alkyl mit 1 bis 5 Kohlenstoffatomen,

$R^3$ ein Wasserstoff oder Alkyl mit 1 bis 5 Kohlenstoffatomen ist,

m 1 oder 2 ist,

$R^4$ ein Cycloalkylrest $-CH(CH_2)n$ ist, in dem n eine ganze Zahl von 2 bis 5 ist,

R5 ein Phenylrest, der einfach, zwei- oder dreifach substituiert sein kann durch Halogen, Alkylreste mit 1 bis 5 Kohlenstoffatomen oder Alkoxyreste mit bis zu 3 Kohlenstoffatomen, die ggf. durch Halogen substituiert sein können, wobei die Substituenten gleich oder verschieden sein können, oder ein Thienylrest ist,

Q eine Ethylen-1,2-diyl-Gruppe (-CH=CH-) oder eine Cyclopropan-1,2-diyl-Gruppe

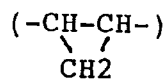

ist,

und ihre Säureadditionssalze.

2. N-Cycloalkylalkylamine nach Anspruch 1, dadurch gekennzeichnet, daß $R^1$ Thienyl ist.

3. N-Cycloalkylalkylamine nach Anspruch 1 oder 2, dadurch daß $R^5$ Phenyl ist.

4. N-Cycloalkylalkylamine nach einem der Ansprüche 1 bis 3, dadurch gekennzeichnet, daß $R^2$ Ethyl ist.

5. N-Cycloalkylalkylamine nach einem der Ansprüche 1 bis 4, dadurch gekennzeichnet, daß $R^3$ Methyl ist.

6. N-Cycloalkylalkylamine nach einem der Ansprüche 1 bis 5, dadurch gekennzeichnet, daß m gleich 1 und $R^4$ ein Cyclopropyl ist.

7. Trans-1-[2-(N-Cyclopropylmethyl-N-methyl)-amino-2-(2-thienyl)-butyl]-2-phenyl-cyclopropan und seine Säureadditionssalze.

8. 4-(N-Cyclopropylmethyl-N-methyl)-amino-1-phenyl-4-(2-thienyl)-hex-1-en und seine Additionssalze.

9. Verfahren zur Herstellung von N-Cycloalkylalkylaminen (I) nach einem der Ansprüche 1 bis 8, dadurch gekennzeichnet,
   - daß man zur Herstellung einer Verbindung (II) der allgemeinen Formel (I), in der $R^3$ Wasserstoff ist,
     i) ein N-carboxamid-Zwischenprodukt (IV)

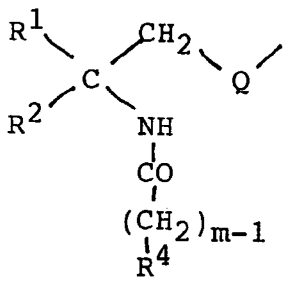

(IV)

durch ein Metallhydrid (Hm) der Formel

$$M^1(t)M^2H_{(r)}Rx_{(s)} \qquad (Hm)$$

reduziert, in der

$M^1$ ein Alkaliatom wie Lithium oder Natrium ist, dessen Kennzahl (t) die Werte 0 oder 1 hat,

$M^2$ ein Element der Gruppe III des periodischen Elementesystems und vorzugsweise Bor oder Aluminium ist, wobei (r) die Kennzahl der Anzahl Wasserstoffatome des Hydrids ist und den Wert 1, 2, 3 oder 4 hat,

Rx eine Carbonitrilgruppe, ein Alkylrest mit 1 bis 5 Kohlenstoffatomen oder ein Alkoxyrest mit bis zu 3 Kohlenstoffatomen, das ggf. durch Halogenatome substituiert ist, wobei die Kennzahl (s) den Wert 0, 1, 2 oder 3 hat,

die Kennzahlen (t), (r) und (s) die Gleichung (r) + (s) - (t) = 3 erfüllen, wobei diese Reduktion vorzugsweise durch ein Hydrid (Hm.2) durchgeführt wird, in dem $M^2$ Aluminium oder auch Bor ist, wenn (r) den Wert 3 und (s) und (t) den Wert 0 haben,

ii) ein Amin (V) der Formel

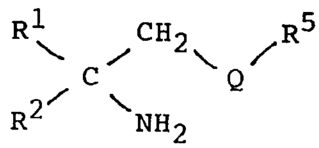

(V)

durch ein Alkylhalogenid $R^4$-$(CH_2)_m$-Z2 alkyliert, in dem Z2 ein Halogen wie Chlor, Brom oder Jod ist,

   - daß man zur Herstellung eines N-Cycloalkylalkylamins (III) der allgemeinen Formel (1), in der $R^3$ ein Alkyl mit 1 bis 5 Kohlenstoffatomen ist,
     i) eine reduzierende Alkylierung einer erfindungsgemäßen Verbindung (II) vorgenommen wird, die darin besteht, daß man diese Verbindung (II) mit einem Aldehyd $R^6$-CHO, in dem $R^6$ das unmittelbar dem Rest $R^3$ vorausgehende kürzere Kohlenstoffhomologe ist ($R^3$ = $CH_2$-$R^6$), in Anwesenheit eines Reduktionsmittels wie eines Metall- oder Organometallhydrids (Hm.3) der oben definierten Formel (Hm), in der $M^2$ Bor und Rx bevorzugt eine Carbonitrilgruppe ist, reagieren läßt, oder
     ii) durch ein oben definiertes Metallhydrid (Hm.2) ein N-carboxamid-Zwischenprodukt (VIII)

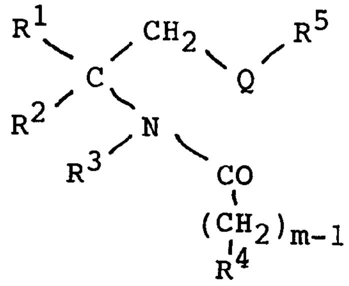

(VIII)

reduziert,

iii) oder ein Alkylmagnesiumreagenz $R^2MgZ3$, in dem Z3 ein Halogen wie Chlor, Brom oder Jod ist, mit einem Aminonitrilzwischenprodukt (IX )

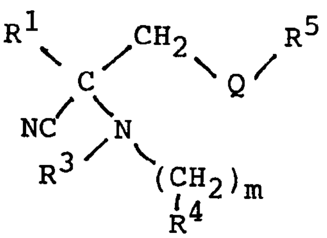

(IX)

reagieren läßt,

iiii) oder durch ein Alkylhalogenid $R^4$-$(CH_2)_m$-Z2, in dem Z2 Chlor, Brom oder Jod ist, ein Amin (VII) der Formel

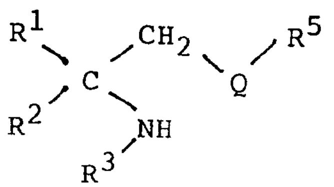

(VII)

alkyliert.

**10.** Arzneimittel, dadurch gekennzeichnet, daß es ein N-Cycloalkylalkylamin der Formel (I) gemäß einem der Ansprüche 1 bis 8 enthält.

**11.** Verwendung eines N-Cycloalkylalkylamins der Formel (I) gemäß einem der Ansprüche 1 bis 8 für die Herstellung eines Arzneimittels zur Behandlung von neurologischen und/oder psychischen Erkrankungen.

**12.** Verwendung eines N-Cycloalkylalkylamins der Formel (I) gemäß einem der Ansprüche 1 bis 8 zur Herstellung eines Arzneimittels zur Behandlung von Dysfunktionen des MagenDarm-Traktes.

**13.** Verwendung eines N-Cycloalkylalkylamins der Formel (I) gemäß einem der Ansprüche 1 bis 8 zur Herstellung eines Arzneimittels zur Behandlung von Magen- und/oder Magen-Zwölffingerdarm-Ulzerationen.

**Patentansprüche für folgenden Vertragsstaat : ES**

**1.** Verfahren zur Herstellung von N-Cycloalkylalkylaminen der allgemeinen Formel (I)

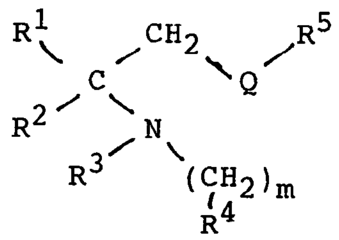

(I)

in der

$R^1$ ein aromatischer Thienyl- oder Furylrest oder ein Phenylrest ist, sofern Q eine Cyclopropan-1,2-diyl-Gruppe

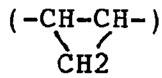

ist,

$R^2$ ein Alkyl mit 1 bis 5 Wasserkohlenstoffatomen ist,

$R^3$ ein Wasserstoff oder Alkyl mit 1 bis 5 Kohlenstoffatomen ist,

m 1 oder 2 beträgt,

$R^4$ ein Cycloalkylrest $-CH(CH_2)_n$ ist, in dem n eine ganze Zahl von 2 bis 5 ist,

R5 ein Phenylrest ist, der einfach, zwei- oder dreifach substituiert sein kann durch Halogen, Alkylreste mit 1 bis 5 Kohlenstoffatomen oder Alkoxyreste mit bis zu 3 Kohlenstoffatomen, die ggf. durch Halogen substituiert sein können, wobei die Substituenten gleich oder verschieden sein können,
oder ein Thienylrest ist,

Q eine Ethylen-1,2-diyl-Gruppe (-CH=CH-) oder eine Cyclopropan-1,2-diyl-Gruppe

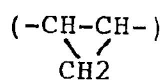

ist,

und ihrer Säureadditionssalze, dadurch gekennzeichnet,

- daß man zur Herstellung einer Verbindung (II) der allgemeinen Formel (1), in der $R^3$ Wasserstoff ist,
i) ein N-Carboxamid-Zwischenprodukt (IV)

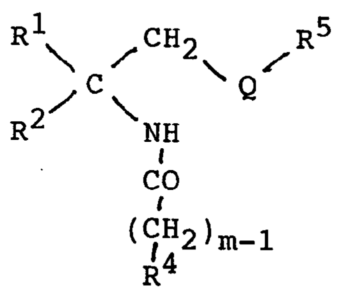

(IV)

durch ein Metallhydrid (Hm) der Formel

$$M^1_{(t)}M^2H_{(r)}Rx_{(s)} \qquad (Hm)$$

reduziert, in der

$M^1$ ein Alkaliatom wie Lithium oder Natrium ist, dessen Kennzahl (t) die Werte 0 oder 1 hat,

$M^2$ ein Element der Gruppe III des periodischen Elementesystems und vorzugsweise Bor oder Aluminium ist, wobei (r) die Kennzahl der Anzahl Wasserstoffatome des Hydrids ist und den Wert 1, 2, 3 oder 4 hat,

Rx eine Carbonitrilgruppe, ein Alkylrest mit 1 bis 5 Kohlenstoffatomen oder ein Alkoxyrest mit bis zu 3 Kohlenstoffatomen ist, der ggf. durch Halogenatome substituiert ist, wobei die Kennzahl (s) den Wert 0, 1, 2 oder 3 hat,

die Kennzahlen (t), (r) und (s) die Gleichung (r) + (s) - (t) = 3 erfüllen, wobei diese Reduktion vorzugsweise durch ein Hydrid (Hm.2) durchgeführt wird, in dem $M^2$ Aluminium oder auch Bor ist, wenn (r) den Wert 3 und (s) und (t) den Wert 0 haben,

ii) ein Amin (V) der Formel

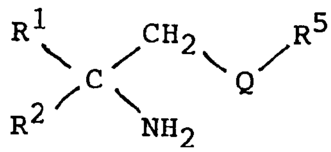

(V)

durch ein Alkylhalogenid $R^4$-$(CH_2)_m$-Z2 alkyliert , in dem Z2 ein Halogen wie Chlor, Brom oder Jod ist,

- daß man zur Herstellung eines N-Cycloalkylalkylamins (III) der allgemeinen Formel (I), in der $R^3$ ein Alkyl mit 1 bis 5 Kohlenstoffatomen ist,

i) eine reduzierende Alkylierung einer erfindungsgemäßen Verbindung (II) vornimmt, die darin besteht, daß man diese Verbindung (II) mit einem Aldehyd $R^6$-CHO, in dem $R^6$ das unmittelbar dem Rest $R^3$ vorausgehende kürzere Kohlenstoffhomologe ist ($R^3$ = $CH_2$-$R^6$), in Anwesenheit eines Reduktionsmittels wie eines Metall- oder Organometallhydrids (Hm.3) der oben definierten Formel (Hm), in der $M^2$ Bor und Rx bevorzugt eine Carbonitrilgruppe ist, reagieren läßt, oder

ii) durch ein oben definiertes Metallhydrid (Hm.2) ein N-carboxamid-Zwischenprodukt (VIII)

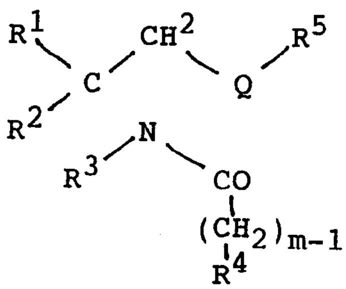

(VIII)

reduziert,

iii) oder ein Alkylmagnesiumreagenz $R^2$MgZ3, in dem Z3 ein Halogen wie Chlor, Brom oder Jod ist, mit einem Aminonitrilzwischenprodukt (IX)

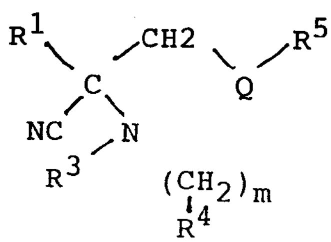

(IX)

reagieren läßt,

iiii) oder durch ein Alkylhalogenid $R^4$-$(CH_2)_m$-Z2, in dem Z2 Chlor, Brom oder Jod ist, ein Amin (VII) der Formel

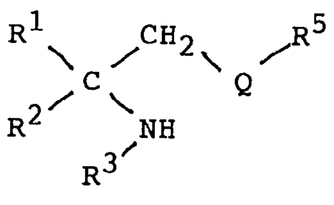

(VII)

alkyliert.

2. Verfahren nach Anspruch 1, dadurch gekennzeichnet, daß $R^1$ Thienyl ist.

3. Verfahren nach Anspruch 1 oder 2, dadurch gekennzeichnet, daß $R^5$ Phenyl ist.

4. Verfahren nach Anspruch 1 bis 3, dadurch gekennzeichnet, daß $R^2$ Ethyl ist.

5. Verfahren nach einem der Ansprüche 1 bis 4, dadurch gekennzeichnet, daß $R^3$ Methyl ist.

6. Verfahren nach einem der Ansprüche 1 bis 5, dadurch gekennzeichnet, daß m den Wert 1 hat und $R^4$ ein Cyclopropyl ist.

7. Verfahren nach Anspruch 1, dadurch gekennzeichnet, daß $R^1$ 2-Thienyl, $R^2$ Ethyl, $R^3$ Methyl, m gleich 1, $R^4$ Cyclopropyl, $R^5$ Phenyl und Q Cyclopropan-1,2-diyl ist.

8. Verfahren nach Anspruch 1, dadurch gekennzeichnet, daß $R^1$ 2-Thienyl, $R^2$ Ethyl, $R^3$ Methyl, m gleich 1, $R^4$ Cyclopropyl, $R^5$ Phenyl und Q Ethylen-1,2-diyl ist.

9. Verfahren zur Herstellung eines Arzneimittels, dadurch gekennzeichnet, daß ein N-Cycloalkylalkylamin der Formel (I) gemäß einem der Ansprüche 1 bis 8 mit einer pharmazeutisch akzeptablen Grundsubstanz gemischt wird.

10. Verwendung eines N-Cycloalkylalkylamins der Formel (I) gemäß einem der Ansprüche 1 bis 8 zur Herstellung eines Arzneimittels zur Behandlung von neurologischen und/oder psychischen Erkrankungen.

11. Verwendung eines N-Cycloalkylalkylamins der Formel (I) gemäß einem der Ansprüche 1 bis 8 zur Herstellung eines Arzneimittels zur Behandlung von Dysfunktionen des Magen-Darm-Traktes.

12. Verwendung eines N-Cycloalkylalkylamins der Formel (I) gemäß einem der Ansprüche 1 bis 8 zur Herstellung eines Arzneimittels zur Behandlung von Magen- und/oder Magen-Zwölffingerdarm-Ulzerationen.

**Patentansprüche für folgenden Vertragsstaat : GR**

1. N-Cycloalkylalkylamine der allgemeinen Formel (I)

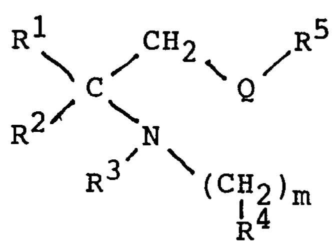

in der

$R^1$ ein aromatischer Thienyl- oder Furylrest oder ein Phenylrest ist, sofern Q eine Cyclopropan-1,2 diyl-Gruppe

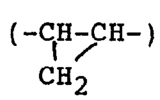

ist,

$R^2$ ein Alkyl mit 1 bis 5 Kohlenstoffatomen ist,

$R^3$ ein Wasserstoff oder Alkyl mit 1 bis 5 Kohlenstoffatomen ist,

m 1 oder 2 ist,

$R^4$ ein Cycloalkylrest $-CH(CH_2)_n$ ist, in dem n eine ganze Zahl von 2 bis 5 ist,

$R^5$ ein Phenylrest, der einfach, zwei- oder dreifach substituiert sein kann durch Halogen, Alkylreste mit 1 bis 5 Kohlenstoffatomen oder Alkoxyreste mit bis zu 3 Kohlenstoffatomen, die ggf. durch

Halogen substituiert sein können, wobei die Substituenten gleich oder verschieden sein können, oder ein Thienylradikal ist,

Q eine Ethylen-1-2-diyl-Gruppe (-CH=CH-) oder eine Cyclopropan-1,2-diyl-Gruppe

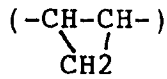

ist,

und ihre Säureadditionssalze.

2. N-Cycloalkylalkylamine nach Anspruch 1, dadurch gekennzeichnet, daß $R^1$ Thienyl ist.

3. N-Cycloalkylalkylamine nach Anspruch 1 oder 2, dadurch daß $R^5$ Phenyl ist.

4. N-Cycloalkylalkylamine nach einem der Ansprüche 1 bis 3, dadurch gekennzeichnet, daß $R^2$ Ethyl ist.

5. N-Cycloalkylalkylamine nach einem der Ansprüche 1 bis 4, dadurch gekennzeichnet, daß $R^3$ Methyl ist.

6. N-Cycloalkylalkylamine nach einem der Ansprüche 1 bis 5, dadurch gekennzeichnet, daß m gleich 1 und $R^4$ ein Cyclopropyl ist.

7. Trans-1-[2-(N-Cyclopropylmethyl-N-methyl)-amino-2-(2-thienyl)-butyl]-2-phenyl-cyclopropan und seine Säureadditionssalze.

8. 4-(N-Cyclopropylmethyl-N-methyl)-amino-1-phenyl-4-(2-thienyl)-hex-1-en und seine Säureadditionssalze.

9. Verfahren zur Herstellung von N-Cycloalkylalkylaminen (I) nach einem der Ansprüche 1 bis 8, dadurch gekennzeichnet,

- daß man zur Herstellung einer Verbindung (II) der allgemeinen Formel (I), in der $R^3$ Wasserstoff ist, i) ein N-carboxamid-Zwischenprodukt (IV)

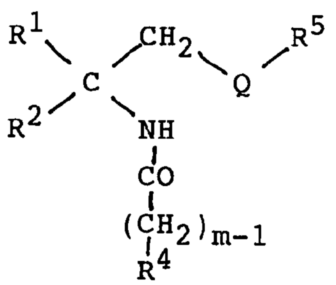

(IV)

durch ein Metallhydrid (Hm) der Formel

$$M^1_{(t)}M^2H_{(r)}Rx_{(s)} \quad (Hm)$$

reduziert, in der

$M^1$ ein Alkaliatom wie Lithium oder Natrium ist, dessen Kennzahl (t) die Werte 0 oder 1 hat,

$M^2$ ein Element der Gruppe III des periodischen Elementesystems und vorzugsweise Bor oder Aluminium ist, wobei (r) die Kennzahl der Anzahl Wässerstoffatome des Hydrids ist und den Wert 1, 2, 3 oder 4 hat,

Rx eine Carbonitrilgruppe, ein Alkylrest mit 1 bis 5 Kohlenstoffatomen oder ein Alkoxyrest mit bis zu 3 Kohlenstoffatomen, das ggf. durch Halogenatome substituiert ist, wobei die Kennzahl (s) den Wert 0, 1, 2 oder 3 hat,

die Kennzahlen (t), (r) und (s) die Gleichung (r) + (s) - (t) = 3 erfüllen, wobei diese Reduktion vorzugsweise durch ein Hydrid (Hm.2) durchgeführt wird, in dem $M^2$ Aluminium oder auch Bor ist, wenn (r) den Wert 3 und (s) und (t) den Wert 0 haben,

ii) ein Amin (V) der Formel

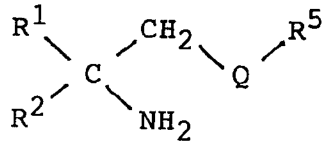

(V)

durch ein Alkylhalogenid $R^4$-$CH_2)_m$-Z2 alkyliert, in dem Z2 ein Halogen wie Chlor, Brom oder Jod ist,

- daß man zur Herstellung eines N-Cycloalkylalkylamins (III) der allgemeinen Formel (I), in der $R^3$ ein Alkyl mit 1 bis 5 Kohlenstoffatomen ist,

  i) eine reduzierende Alkylierung einer erfindungsgemäßen Verbindung (II) vornimmt, die darin besteht, daß man diese Verbindung (II) mit einem Aldehyd $R^6$-CHO, in dem $R^6$ das unmittelbar dem Rest $R^3$ vorausgehende kürzere Kohlenstoffhomologe ist ($R^3$ = $CH_2$-$R^6$), in Anwesenheit eines Reaktionsmittels wie eines Metall- oder Organometallhydrids (Hm.3) der oben definierten Formel (Hm), in der $M^2$ Bor und Rx bevorzugt eine Carbonitrilgruppe ist, reagieren läßt, oder

  ii) durch ein oben definiertes Metallhydrid (Hm.2) ein N-Carboxamid-Zwischenprodukt (VIII)

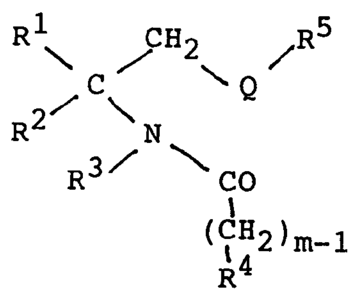

(VIII)

  reduziert,

  iii) oder ein Alkylmagnesiumreagenz $R^2$MgZ3, in dem Z3 ein Halogen wie Chlor, Brom oder Jod ist, mit einem Aminonitrilzwischenprodukt (IX)

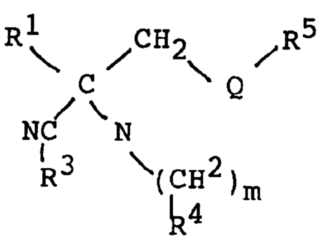

(IX)

  reagieren läßt,

  iiii) oder durch ein Alkylhalogenid $R^4$-(CH2)m-Z2, in dem Z2 Chlor, Brom oder Jod ist, ein Amin (VII) der Formel

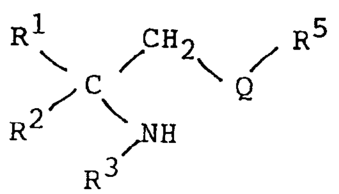

(VII)

  alkyliert.

**10.** Verfahren zur Herstellung eines Arzneimittels, dadurch gekennzeichnet, daß ein N-Cycloalkylalkylamin der Formel (I) gemäß einem der Ansprüche 1 bis 8 mit einer pharmazeutisch akzeptablen Grundsubstanz gemischt wird.

11. Verwendung eines N-Cycloalkylalkylamins der Formel (I) gemäß einem der Ansprüche 1 bis 8 für die Herstellung eines Arzneimittels zur Behandlung von neurologischen und/oder psychischen Erkrankungen.

12. Verwendung eines N-Cycloalkylalkylamins der Formel (I) gemäß einem der Ansprüche 1 bis 8 zur Herstellung eines Arzneimittels zur Behandlung von Dysfunktionen des MagenDarm-Traktes.

13. Verwendung eines N-Cycloalkylalkylamins der Formel (I) gemäß einem der Ansprüche 1 bis 8 zur Herstellung eines Arzneimittels zur Behandlung von Magen- und/oder Magen-Zwölffingerdarm-Ulzerationen.

## Claims

### Claims for the following Contracting States : AT, BE, CH, DE, DK, FR, GB, IT, LI, LU, NL, SE

1. N-Cycloalkylalkylamines of general formula (I):

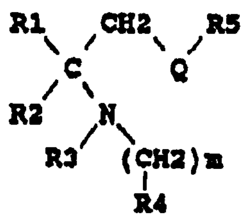

(I)

in which:

R1 is a thienyl or furyl aromatic radical or a phenyl radical with the proviso that Q represents a cyclopropane-1,2-diyl

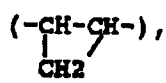

group,

R2 is $C_1$ to $C_5$ alkyl

R3 is hydrogen or $C_1$ to $C_5$ alkyl

m is 1 or 2,

R4 is a cycloalkyl radical $-CH(CH_2)_n$, in which n is an integer from 2 to 5,

R5 is a phenyl radical which may be optionally mono-, di- or trisubstituted with halogens or with $C_1$ to $C_5$ alkyl radicals or with alkoxy radicals up to $C_3$, which may be optionally substituted by halogen atoms and which may be identical or different, or represents a thienyl radical,

Q represents an ethylene-1,2-diyl group (-CH=CH-) or a cyclopropane-1,2-diyl group

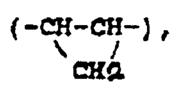

and their addition salts with acids.

2. N-Cycloalkylalkylamines according to Claim 1, characterized in that R1 is thienyl.

3. N-Cycloalkylalkylamines according to Claim 1 or 2, characterized in that R5 is phenyl.

4. N-Cycloalkylalkylamines according to one of Claims 1 to 3, characterized in that R2 is ethyl.

5. N-Cycloalkylalkylamines according to one of Claims 1 to 4, characterized in that R3 is methyl.

6. N-Cycloalkylalkylamines according to one of Claims 1 to 5, characterized in that m is 1 and R4 is cyclopropyl.

7. trans-1-[2-(N-Cyclopropylmethyl-N-methyl)amino-2-(2-thienyl)butyl]-2-phenylcyclopropane and its addition salts with acids.

8. 4-(N-Cyclopropylmethyl-N-methyl)amino-1-phenyl-4-(2-thienyl)-1-hexene and its addition salts with acids.

9. Process for the preparation of N-cycloalkylalkyl- amines (I) as defined in one of Claims 1 to 8, characterized in that it consists
   - for the preparation of a compound (II) of general formula (I) and in which R3 is hydrogen,
     i) in reducing an intermediate N-carboxamide (IV)

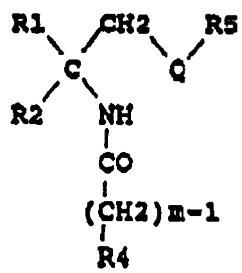

IV

with a metal hydride (Hm) of formula:

$M1_{(t)}M2H_{(r)}Rx_{(s)}$ (Hm)

in which:
M1 represents an alkali metal atom such as lithium or sodium and for which the representative index (t) has the value 0 or 1,
M2 is an element of group III of the Periodic Table of the elements and preferably boron or aluminium, (r) is the index representing the number of hydrogen atoms in the hydride and has the value 1, 2, 3 or 4,
Rx is a carbonitrile group, a $C_1$ to $C_5$ alkyl radical or an alkoxy radical which may be up to $C_3$ and optionally substituted with halogen atoms, for which the representative index (s) has the value 0, 1, 2 or 3,
the above indices (t), (r) and (s) conforming to the relationship: (r) + (s) - (t) = 3, this reduction preferably being carried out by a hydride (Hm.2) in which M2 is aluminium or else boron when (r) is 3 and (s) and (t) have the value 0,
ii) in alkylating an amine (V) of formula

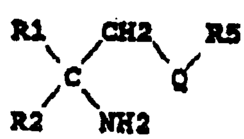

(V)

with an alkyl halide $R4-(CH_2)_m-Z2$ in which Z2 is a halogen such as chlorine, bromine or iodine,
   - for the preparation of an N-cycloalkylalkylamine (III) of general formula (I) in which R3 is $C_1$ to $C_5$ alkyl
     i) in performing a reductive alkylation of a compound (II) of the invention, which consists in reacting this compound (II) with an aldehyde R6-CHO in which R6 is the immediately lower carbon homologue of the radical R3 (R3 = $CH_2$-R6), in the presence of a reducing agent such as a metallic or organometallic hydride (Hm.3) of formula (Hm) defined above and in which M2 is boron and, preferably, Rx is a carbonitrile group, or
     ii) in reducing, with a previously defined metal hydride (Hm.2), an intermediate N-carboxamide (VIII)

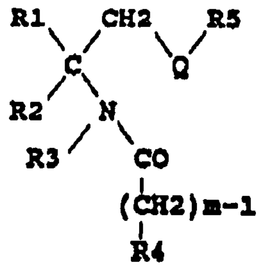

(VIII)

iii) or in reacting an organomagnesium reagent R2MgZ3 in which Z3 is a halogen such as chlorine, bromine or iodine with an aminonitrile inter- mediate (IX)

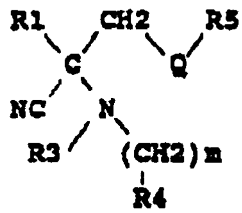

(IX)

iv) or else in alkylating with an alkyl halide R4-$(CH_2)_m$-Z2 in which Z2 is chlorine, bromine or iodine an amide (VII) of formula

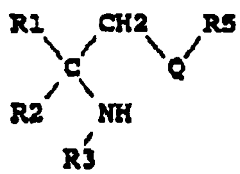

(VII)

10. Medicament, characterized in that it comprises an N-cycloalkylalkylamine of formula (I) as defined in one of Claims 1 to 8.

11. Use of an N-cycloalkylalkylamine of formula (I) as defined in one of Claims 1 to 8, for the production of a medicament intended for the treatment of neurological and/or psychological complaints.

12. Use of an N-cycloalkylalkylamine of formula (I) as defined in one of Claims 1 to 8, for the production of a medicament intended for the treatment of dysfunctions of the gastrointestinal tract.

13. Use of an N-cycloalkylalkylamine of formula (I) as defined in one of Claims 1 to 8, for the production of a medicament intended for the treatment of gastric ulcers and/or gastroduodenal ulcers.

**Claims for the following Contracting State : ES**

1. Process for the preparation of N-cycloalkylamines of general formula (I) :

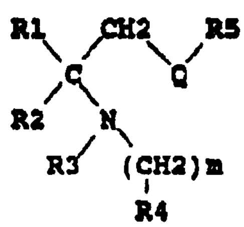

(I)

in which:

R1 is a thienyl or furyl aromatic radical or a phenyl radical with the proviso that Q represents a cyclopropane-1,2-diyl

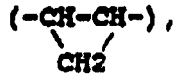

group,

R2 is $C_1$ to $C_5$ alkyl

R3 is hydrogen or $C_1$ to $C_5$ alkyl

m is 1 or 2,

R4 is a cycloalkyl radical $-CH(CH_2)_n$, in which n is an integer from 2 to 5,

R5 is a phenyl radical which may be optionally mono-, di- or trisubstituted with halogens or with $C_1$ to $C_5$ alkyl radicals or with alkoxy radicals up to $C_3$, which may be optionally substituted by halogen atoms and which may be identical or different, or represents a thienyl radical,

Q represents an ethylene-1,2-diyl group (-CH=CH-) or a cyclopropane-1,2-diyl group

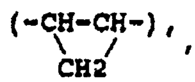

and their addition salts with acids,

characterized in that it consists

- for the preparation of a compound (II) of general formula (I) and in which R3 is hydrogen,

i) in reducing an intermediate N-carboxamide (IV)

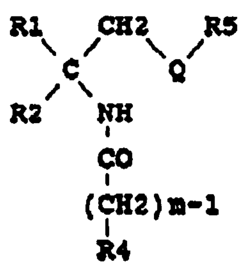

(IV)

with a metal hydride (Hm) of formula:

$$M1_{(t)}M2H_{(r)}Rx_{(s)} \qquad (Hm)$$

in which:

M1 represents an alkali metal atom such as lithium or sodium and for which the representative index (t) has the value 0 or 1,

M2 is an element of group III of the Periodic Table of the elements and preferably boron or aluminium, (r) is the index representing the number of hydrogen atoms in the hydride and has the value 1, 2, 3 or 4,

Rx is a carbonitrile group, a $C_1$ to $C_5$ alkyl radical or an alkoxy radical which may be up to $C_3$ and optionally substituted with halogen atoms, for which the representative index (s) has the value 0, 1, 2 or 3,

the above indices (t), (r) and (s) conforming to the relationship: (r) + (s) - (t) = 3, this reduction preferably being carried out by a hydride (Hm.2) in which M2 is aluminium or else boron when (r) is 3 and (s) and (t) have the value 0,

ii) in alkylating an amine (V) of formula

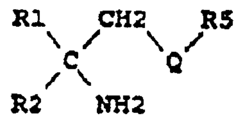

(V)

with an alkyl halide R4-$(CH_2)_m$-Z2 in which Z2 is a halogen such as chlorine, bromine or iodine,

- for the preparation of an N-cycloalkylalkylamine (III) of general formula (I) in which R3 is $C_1$ to $C_5$ alkyl

  i) in performing a reductive alkylation of a compound (II) of the invention, which consists in reacting this compound (II) with an aldehyde R6-CHO in which R6 is the immediately lower carbon homologue of the radical R3 (R3 = $CH_2$-R6), in the presence of a reducing agent such as a metallic or organometallic hydride (Hm.3) of formula (Hm) defined above and in which M2 is boron and, preferably, Rx is a carbonitrile group, or

  ii) in reducing, with a previously defined metal hydride (Hm.2), an intermediate N-carboxamide (VIII)

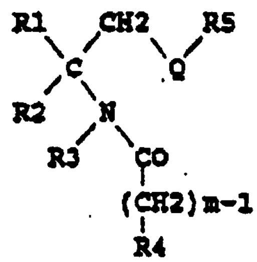

(VIII)

  iii) or in reacting an organomagnesium reagent R2MgZ3 in which Z3 is a halogen such as chlorine, bromine or iodine with an aminonitrile intermediate (IX)

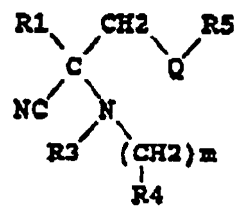

(IX)

  iv) or else in alkylating with an alkyl halide R4-$(CH_2)_m$-Z2 in which Z2 is chlorine, bromine or iodine an amide (VII) of formula

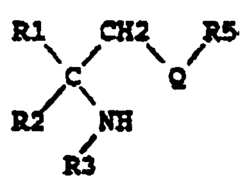

(VII)

**2.** Process according to Claim 1, characterized in that R1 is thienyl.

**3.** Process according to Claim 1 or 2, characterized in that R5 is phenyl.

**4.** Process according to one of Claims 1 to 3, characterized in that R2 is ethyl.

**5.** Process according to one of Claims 1 to 4, characterized in that R3 is methyl.

**6.** Process according to one of Claims 1 to 5, characterized in that m is 1 and R4 is cyclopropyl.

7. Process according to Claim 1, characterized in that R1 is 2-thienyl, R2 is ethyl, R3 is methyl, m is equal to 1, R4 is cyclopropyl, R5 is phenyl and Q is cyclopropane-1,2-diyl.

8. Process according to Claim 1, characterized in that R1 is 2-thienyl, R2 is ethyl, R3 is methyl, m is equal to 1, R4 is cyclopropyl, R5 is phenyl and Q is ethylene-1,2-diyl.

9. Process for the preparation of a medicament, characterized in that it consists in mixing an N-cycloalkylalkylamine of formula (I) as defined in one of Claims 1 to 8 with a pharmaceutically acceptable excipient.

10. Use of an N-cycloalkylalkylamine of formula (I) as defined in one of Claims 1 to 8, for the production of a medicament intended for the treatment of neurological and/or psychological complaints.

11. Use of an N-cycloalkylalkylamine of formula (I) as defined in one of Claims 1 to 8, for the production of a medicament intended for the treatment of dysfunctions of the gastrointestinal tract.

12. Use of an N-cycloalkylalkylamine of formula (I) as defined in one of Claims 1 to 8, for the production of a medicament intended for the treatment of gastric ulcers and/or gastroduodenal ulcers.

**Claims for the following Contracting State : GR**

1. N-cycloalkylalkylamines of general formula (I):

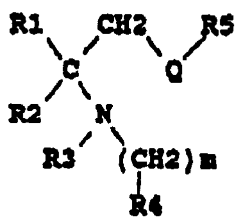

(I)

in which:

R1 is a thienyl or furyl aromatic radical or a phenyl radical with the proviso that Q represents a cyclopropane-1,2-diyl

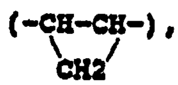

group,

R2 is $C_1$ to $C_5$ alkyl

R3 is hydrogen or $C_1$ to $C_5$ alkyl

m is 1 or 2,

R4 is a cycloalkyl radical $-CH(CH_2)_n$, in which n is an integer from 2 to 5,

R5 is a phenyl radical which may be optionally mono-, di- or trisubstituted with halogens or with $C_1$ to $C_5$ alkyl radicals or with alkoxy radicals up to $C_3$, which may be optionally substituted by halogen atoms and which may be identical or different, or represents a thienyl radical,

Q represents an ethylene-1,2-diyl group (-CH=CH-) or a cyclopropane-1,2-diyl group

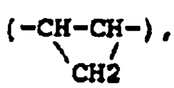

and their addition salts with acids.

2. N-Cycloalkylalkylamines according to Claim 1, characterized in that R1 is thienyl.

3. N-Cycloalkylalkylamines according to Claim 1 or 2, characterized in that R5 is phenyl.

4. N-Cycloalkylalkylamines according to one of Claims 1 to 3, characterized in that R2 is ethyl.

5. N-Cycloalkylalkylamines according to one of Claims 1 to 4, characterized in that R3 is methyl.

6. N-Cycloalkylalkylamines according to one of Claims 1 to 5, characterized in that m is 1 and R4 is cyclopropyl.

7. trans-1-[2-(N-Cyclopropylmethyl-N-methyl)amino-2-(2-thienyl)butyl]-2-phenylcyclopropane and its addition salts with acids.

8. 4-(N-Cyclopropylmethyl-N-methyl)amino-1-phenyl-4-(2-thienyl)-1-hexene and its addition salts with acids.

9. Process for the preparation of N-cycloalkylalkylamines (I) as defined in one of Claims 1 to 8, characterized in that it consists
   - for the preparation of a compound (II) of general formula (I) and in which R3 is hydrogen,
     i) in reducing an intermediate N-carboxamide (IV)

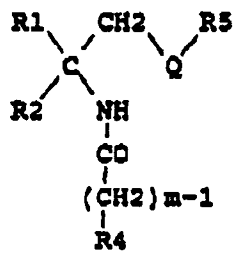

(IV)

with a metal hydride (Hm) of formula:

$$M1_{(t)}M2H_{(r)}Rx_{(s)} \qquad (Hm)$$

in which:
M1 represents an alkali metal atom such as lithium or sodium and for which the representative index (t) has the value 0 or 1,
M2 is an element of group III of the Periodic Table of the elements and preferably boron or aluminium, (r) is the representative index of the number of hydrogen atoms in the hydride and has the value 1, 2, 3 or 4,
Rx is a carbonitrile group, a $C_1$ to $C_5$ alkyl radical or an alkoxy radical which may be up to $C_3$ and optionally substituted with halogen atoms, for which the representative index (s) has the value 0, 1, 2 or 3,
the above indices (t), (r) and (s) conforming to the relationship: (r) + (s) (t) = 3, this reduction preferably being carried out by a hydride (Hm.2) in which M2 is aluminium or else boron when (r) is 3 and (s) and (t) have the value 0,
ii) in alkylating an amine (V) of formula

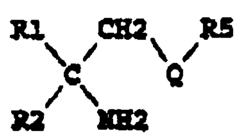

(V)

with an alkyl halide $R4-(CH_2)_m-Z2$ in which Z2 is a halogen such as chlorine, bromine or iodine,
   - for the preparation of an N-cycloalkylalkylamine (III) of general formula (I) in which R3 is $C_1$ to $C_5$ alkyl
     i) in performing a reductive alkylation of a compound (II) of the invention, which consists in reacting this compound (II) with an aldehyde R6-CHO in which R6 is the immediately lower carbon homologue of the radical R3 ($R3 = CH_2-R6$), in the presence of a reducing agent such as a metallic or organometallic hydride (Hm.3) of formula (Hm) defined above and in which M2 is boron and, preferably, Rx is a carbonitrile group, or

ii) in reducing, with a previously defined metal hydride (Hm.2), an intermediate N-carboxamide (VIII)

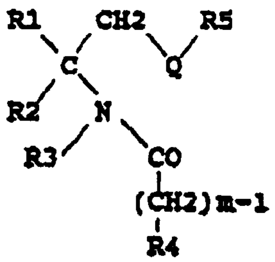

(VIII)

iii) or in reacting an organomagnesium reagent R2MgZ3 in which Z3 is a halogen such as chlorine, bromine or iodine with an aminonitrile inter- mediate (IX)

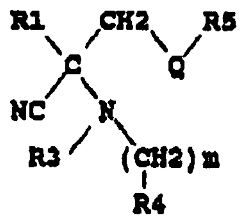

(IX)

iiii) or else in alkylating with an alkyl halide R4-$(CH_2)_m$-Z2 in which Z2 is chlorine, bromine or iodine an amide (VII) of formula

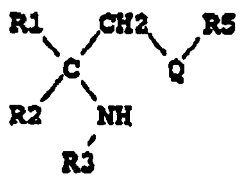

(VII)

**10.** Process for the preparation of a medicament, charac- terized in that it consists in mixing an N-cyclo- alkylalkylamine of formula (I) as defined in one of Claims 1 to 8 with a pharmaceutically acceptable excipient.

**11.** Use of an N-cycloalkylalkylamine of formula (I) as defined in one of Claims 1 to 8, for the production of a medicament intended for the treatment of neurological and/or psychological complaints.

**12.** Use of an N-cycloalkylalkylamine of formula (I) as defined in one of Claims 1 to 8, for the production of a medicament intended for the treatment of dysfunctions of the gastrointestinal tract.

**13.** Use of an N-cycloalkylalkylamine of formula (I) as defined in one of Claims 1 to 8, for the production of a medicament intended for the treatment of gastric ulcers and/or gastroduodenal ulcers.